# EUROPEAN PATENT SPECIFICATION

(11) **EP 2 040 674 B1**
(45) Date of publication and mention of the grant of the patent: **07.07.2010**
(21) Application number: 07725375.5
(22) Date of filing: 18.05.2007
(51) Int. Cl.: A61K 9/133, A61P 19/02, A61K 31/519, A61K 31/337

(54) **CATIONIC LIPOSOMAL PREPARATIONS FOR THE TREATMENT OF RHEUMATOID ARTHRITIS**
KATIONISCHE LIPOSOMALE ZUBEREITUNGEN ZUR BEHANDLUNG VON RHEUMATOIDER ARTHRITIS
PRÉPARATIONS LIPOSOMALES CATIONIQUES POUR LE TRAITEMENT DE LA POLYARTHRITE RHUMATOÏDE

(30) Priority: 18.05.2006 US 801079 P; 09.01.2007 US 879320 P
(43) Date of publication of application: 01.04.2009
(73) Proprietor: MediGene AG, 82152 Planegg (DE); Ludwig-Maximilians-Universität, 80539 München (DE)
(72) Inventor: FUNK, Martin, 86949 Windach (DE); SCHULZE, Brita, 82432 Walchensee (DE); GUENZI, Eric, 85221 Dachau (DE); MICHAELIS, Uwe, 82362 Weilheim (DE); BOHNENKAMP, Hermann, 81373 München (DE); EICHHORN, Martin, 81371 München (DE); SCHMITT-SODY, Marcus, 82237 München (DE)
(74) Representative: Weiss, Wolfgang
(86) International application number: PCT/EP2007/004467
(87) International publication number: WO 2007/134819

(56) References cited:
- EP-A- 1 374 864
- WO-A-99/33493
- WO-A-99/54344
- WO-A-2004/002455
- WO-A-2005/039533
- WO-A2-01/82899
- US-A1- 2005 143 336
- US-B1- 6 852 334
- TYRRELL D.A. ET AL.: "The effect of serum protein fractions on liposome-cell interactions in cultured cells and the perfused rat liver" BIOCHIMICA ET BIOPHYSICA ACTA, vol. 497, no. 2, 1977, pages 469-480, XP002452459
- KAYE S.B. ET AL.: "The effect of liposome (phospholipid vesicle) entrapment of actinomycin D and methotrexate on the in vivo treatment of sensitive and resistant solid murine tumours" EUROPEAN JOURNAL OF CANCER, vol. 17, no. 3, 1981, pages 279-289, XP002452460
- THURSTON G. ET AL.: "CATIONIC LIPOSOMES TARGET ANGIOGENIC ENDOTHELIAL CELLS IN TUMORS AND CHRONIC INFLAMMATION IN MICE" JOURNAL OF CLINICAL INVESTIGATION, NEW YORK, NY, US, vol. 101, no. 7, 1 April 1998 (1998-04-01), pages 1401-1413, XP000863045 ISSN: 0021-9738

## Description

The present invention refers to the treatment of inflammatory and/or autoimmune disorders, particularly of arthritic disorders such as rheumatoid arthritis, osteoarthritis, psoriatic arthritis, spondyloarthropathies, ankylosing spondylitis or morbus Reiter.

### Introduction

Rheumatoid arthritis (RA) is a chronic inflammatory and destructive joint disease affecting 0.5-1.0 % of the population in the industrialized world {Gabriel, 2001 #1}. It commonly leads to significant disability and consequently to a significant reduction of quality of life. If not treated appropriately, RA leads to a reduction of life expectancy {Smolen, 2003 #2}. The disease is a polyarthritis usually involving several joints, most commonly involved are those of hands, feet, knees. RA leads to swelling of the joints, pain caused by the inflammatory processes and finally to a destruction of the joints.

RA is initially triggered by an inflammatory response of the synovial membrane, so called synovitis, followed by a local activation or transendothelial influx of mononuclear cells like T cells, B cells, dendritic cells, plasma cells, macrophages, mast cells and by the formation of new blood vessels. The lining layer of synoviocytes becomes hyperplastic and the synovial membrane expands forming villi. Although rheumatoid arthritis shares the features of infiltration of immune cells and hypoplasia with other inflammatory arthritides, it is a special feature of rheumatoid arthritis that the synovium becomes locally invasive at the synovial interface between cartilage and bone. The invasive front comprising osteoclasts, called pannus, leads to cartilage degradation and bone erosions. Cartilage degradation is performed by enzymes secreted from neutrophils, synoviocytes and chondrocytes.

RA is based on a complex pattern of cellular events starting with T-cells invading the synovial membrane that produce interleukin-2 (IL-2) and interferon-gamma (IFN-gamma) {Steiner, 1999 #3}. These T-cells get further activated by antigen-presenting cells carrying arthritogenic (auto)antigens and activate monocytes, macrophages and synovial fibroblasts by further secretion of IL-2 and IFN-gamma {Stout, 1993 #4} Overproduction of TNF-alpha, IL-1 and IL-6 by T-cell activated macrophages seems to be the pivotal event leading to chronic inflammation. These cytokines induce the expression of various other cytokines and matrix metalloproteinases (MMPs) involved in tissue degradation. TNF-alpha and IL-1 stimulate the formation of osteoclasts that resorb and destroy bone and activate chondrocytes secreting further MMPs {Smolen, 2003 #2}.

In recent years, it has been recognized that the process of angiogenesis, the growth of new blood vessels, plays a crucial role in the course of rheumatoid arthritis. The synovial lining of a normal joint is highly vascularized providing support to the avascular articular cartilage. Normally, vascular proliferation is not observed in healthy joints. During RA increased angiogenesis leads to reorganization of articular blood vessels.

During synovial hyperplasia observed in RA the distance between proliferating cells of the synovial lining and the nearest blood vessels increases. This results in hypoxic conditions and hypoperfusion that, combined with a high metabolic demand from proliferating synoviocytes, leads to a potent angiogenic stimulus. This drives angiogenesis accompanied by further infiltration of monocytes, inflammation and hyperplasia {Paleolog, 2002 #5}. Finally this leads to an increased vascular density in the deeper synovial as well as to growth and invasion of the vascularized pannus into the articular cartilage and subchondral bone, being the prerequisite for endochondral ossification at the osteochondral junction.

Many of the growth factors, cytokines and chemokines found to be elevated in the RA synovium are able to stimulate angiogenesis, in most part indirectly by upregulating the expression of more potent specific angiogenic stimuli. Broad range mitogens like FGF-1, FGF-2, PDGF and HGF, which are capable of promoting angiogenesis, are secreted by macrophages, lining cells and endothelial cells in the RA synovium {Koch, 2000 #6} {Paleolog, 2002 #5}.

In contrast, VEGF is an endothelial specific angiogenic factor that is upregulated by hypoxic conditions as they are found in the RA pannus. VEGF levels are higher in the serum and synovial fluids of RA patients. In addition high VEGF levels at an early disease stage are associated with an increased subsequent damage to joints observed later.

The question, whether the inhibition of the angiogenic process might offer an additional therapeutic concept in RA has been evaluated in animal models. Taxol®, TNP-470 and thalidomide are compounds that exert nonspecific anti-angiogenic (and other) effects that have been shown to inhibit angiogenesis and pannus formation in these models. Blockade of VEGF signaling by soluble form of the Flt-1 VEGF receptor or polyclonal anti-VEGF antibodies reduced disease severity and joint destruction in murine collagen induced arthritis. Indirect evidence from treatment with DMARDs show that compounds like Methotrexate inhibit angiogenesis in experimental systems and that part of the beneficial clinical effect of TNF-alpha in RA may be due to the reduction of blood vessel formation {Paleolog, 2002 #5}

To date, the pharmaceutical therapy of RA is based on two principal approaches. The first treatment option is a symptomatic treatment with corticosteroids or preferably non-steroidal anti-inflammatory drugs (NSAIDs). NSAIDs inhibit the generation of prostaglandin by cyclooxygenases, thus only interfere with a small segment of the inflammatory cascade. This class of drugs neither targets the underlying immuno-inflammatory mechanisms of RA, nor retards joint destruction.

Disease-modifying antirheumatic drugs (DMARDs) are the second major class of drugs currently used in the treatment of RA. DMARDs are agents that interfere with both, the inflammatory and destructive processes of RA, thereby modify the principal course of the disease. If effective, these agents reduce swelling and pain, as well as progression of destructive processes, thereby rendering treatment with other anti-inflammatory agents like NSAIDs or glucocorticoids redundant. DMARDs can be divided into small molecules and biological agents, the latter ones being in the scope of drug development in the recent years, due to the growing understanding of the biological events underlying the disease.

The group of small molecule NSAIDs comprises agents like Methotrexate (MTX), Gold salts, Sulphasalazine, Glucocorticoids, Antimalaria Drugs, Cyclosporine A, Minocycline, Azathioprine and Leflunomide {Smolen, 2003 #2}.

Among these drugs, Methotrexate is the first choice treatment for RA in the United States and Europe, and represents the standard by which new DMARDs are evaluated. MTX is a folate analogue that inhibits dihydrofolate reductase (DHFR), leading to an inhibition of DNA synthesis. Hence, MTX mainly affects proliferating cells, such as malignant cells, and also activated peripheral blood lymphocytes. This anti-proliferative effects on lymphocytes and other proliferating cells in the inflamed joint has been the rationale behind the application of MTX in RA. The broad experience gathered in recent years from clinical data and basic research has suggested that effect of low dose MTX treatment might be more anti-inflammatory than anti-proliferative {Cutolo, 2001 #7}.

Unfortunately the action of MTX does not only facilitate its curative action in RA but also exerts a wide range of undesired side effects, affecting the gastrointestinal, cutaneous, central nervous, hematologic, hepatic, pulmonary and immunologic system. Toxicity, rather than lack of efficacy, is the most common cause of discontinuing MTX therapy {Borchers, 2004 #8}. The relatively short plasma half-live, largely due to fast renal excretion, displays another disadvantage of the treatment with current MTX formulations.

The growing understanding of the molecular basis underlying the disease, especially of the inflammatory process, has driven the development of biological DMARDs as further therapies for RA. Due to its central role in the inflammatory process, TNF-alpha has been an important target of the drug development effort. As a result, antibodies (Infliximab, Adalimumab) and a soluble receptor construct neutralizing the action of TNF-alpha (Etanercept) have been established in the treatment of RA. Other new therapies include an antagonist against the IL-1 receptor as an important pro-inflammatory cytokine.

Further targets of the current drug development process include the matrix metalloproteinases (MMP) which seem to be important in the destructive processes in the inflamed joints, as they are present in increased levels in their active form. Also several components of the signal transduction pathways of TNF-alpha and IL-1 are currently in the focus of development.

The vascular endothelium as the barrier between the blood and the synovium has also been determined as a possible point of intervention, as the monocytes/macrophages and other leucocytes have to migrate across this barrier to exert their crucial role in the inflammatory reactions in RA {Szekanecz, 2000 #9}. This migration process involves the interaction of the leucocytes with the endothelial cells via several cellular adhesion molecules and cell surface receptors. Among those, the αᵥβ₃ integrin was identified as one of the important molecules. αᵥβ₃ integrin modulates the growth, survival and motility of angiogenic endothelial cells {Wilder, 2002 #10}. The interaction of αᵥβ₃ integrin with its ligands like vitronectin, fibronectin and fibrinogen is mediated via the RGD three amino acid sequence motive. It could be shown that an inhibition of αᵥβ₃ integrin by RGD peptides induces apoptosis of endothelial cells and inhibits angiogenesis. The treatment with a cyclic RGD peptide drug lead to the inhibition of of synovial angiogenesis, reduced synovial cell infiltrate, pannus formation and cartilage erosion, resulting in an overall reduction of the severity of arthritis in animal models {Miller, 2000 #11 {Badger, 2001 #12}.

Though DMARDs target the basic processes of RA, remission only occurs in 20-25 % of patients in clinical practice {Smolen, 2003 #2}. Many of the agents currently used in the treatment of RA have a dose-limiting therapeutic index and although new biological treatments have improved efficacy of treatment, these agents still have system safety implications {Garrood, 2006 #13}.

Thus, there is still demand of more efficiant and safe agents and means for the prevention and/or treatment of RA.

### Description of the invention

The underlying problem of the present invention was to provide new means for the treatment of inflammatory and/or autoimmune disorders, particularly of angiogenesis-associated inflammatory and/or autoimmune disorders, more particularly of psoriasis, inflammatory bowel disease and particularly arthritic disorders such as rheumatoid arthritis, osteoarthritis, psoriatic arthritis, spondyloarthropathies, ankylosing spondylitis, Churg-Strauss syndrome, fibromyalgia, giant cell arthritis, gout, Henoch-Schoenlein Purpura, hypersensitivity vasculitis, polyarthritis nodosa, systemic lupus erythematosus, systemic sclerosis, takayasu arthritis, Wegener's granulomatosis or morbus Reiter. The problem is solved by the invention as disclosed herein in its embodiments.

The present invention provides the use of a cationic liposomal preparation having a positive zeta potential and at least one active agent, e.g. a non-nucleotidic agent such as small molecule or polypeptide active agent, for the manufacture of pharmaceutical composition for the prevention and/or treatment of inflammatory and/or autoimmune disorders, particularly of angiogenesis-associated inflammatory and/or autoimmune disorders, more particularly of arthritic disorders such as rheumatoid arthritis, osteoarthritis, psoriatic arthritis, spondyloarthropathies, ankylosing spondylitis or morbus Reiter by systemic administration. Most preferably the pharmaceutical composition is used to treat rheumatoid arthritis in a mammal, e.g. a human patient in need of such a treatment.

Thus, it is an aspect of the current invention to provide a method of treating rheumatoid arthritis and related disorders comprising the systemic administration of said liposomal preparation to a human patient.

The small molecule or polypeptide active agent can be selected from a non-steroidal anti-inflammatory drug, a disease-modifying anti-rheumatic drug, a cytotoxic agent or a cytostatic agent or combinations thereof.

It has been surprisingly found that cationic liposomes having a positive zeta potential can be efficiently targeted to the vascular endothelium of joints in mice with antigen induced arthritis in comparison to untreated mice. In addition, it has been surprisingly found that cationic liposomes show a lower binding to the vascular endothelium of normal mice when compared to the binding of neutral or anionic liposomes to the endothelium of these animals. Neutral or anionic liposomes did not exhibit such a pronounced targeting effect when comparing the endothelium of arthritic mice with untreated mice. In addition it has been surprisingly found that cationic liposomes exhibit a lower binding to the vascular endothelium of normal mice when compard to the binding of neutral or anionic liposomes to the endothelium of these animals. Although targeting of cationic liposomes to angiogenic endothelial cells of a tumor is disclosed in WO 98/40052 by McDonald et al. or in WO 01/82899 by Schulze et al., it could not be predicted that the overall properties of angiogenic endothelial cells in rheumatoid arthritis facilitate targeting of said cationic liposomes also in an inflamed joint. Surprisingly, the targeting effect of cationic liposomes harbouring a positive zeta potential was observed not only in the proliferating angiogenic endothelial cells, but also in non-proliferating endothelial cells activated by TNF-alpha, which is a central mediator of inflammation in RA at the vascular endothelium or the synovial tissue of inflamed joints.

Further, systemic administration of a cationic liposomal preparation comprising at least one active agent, and having a positive zeta potential reduces the infiltration of mononuclear cells into the synovial tissue, which is a hallmark of inflammation, pannus development and cartilage erosion. The administration also reduced the number of osteoclasts in the joint's bones, resulting in a reduced erosion of bone in the course of rheumatoid arthritis. Hence it is another aspect of the present invention to provide a method of preventing and/or inhibiting cartilage and/or bone erosion in the course of inflammatory and/or autoimmune disorders such as rheumatoid arthritis or related disorders, comprising the systemic administration of a cationic liposomal preparation comprising at least one active agent, and having a positive zeta potential.

Accordingly, it is another aspect of the present invention to provide a cationic liposomal preparation comprising at least one active agent, and having a positive zeta potential for the manufacture of a medicament for the prevention and/or treatment of cartilage and/or bone erosion in the course of an inflammatory and/or autoimmune disorder such as rheumatoid arthritis.

The invention also provides a method of selectively delivering at least one active agent to the vascular endothelium of arthritic joints, comprising the systemic administration of a cationic liposomal preparation, said preparation having a positive zeta potential. In a further aspect, the invention relates to a method of selectively delivering at least one active agent to the synovial tissue in arthritic joints, comprising the systemic administration of a cationic liposomal preparation having a positive zeta potential. The method can be utilized to deliver drugs, e.g. DMARDs, to the vasculature of arthitic joints and/or to destroy the angiogenic vasculature of the arthritic joint.

As described above, the invention enables the selective delivery of at least one active agents to the vascular endothelium of arthritic joints. Nonetheless, the effect of this targeting is not only limited to the close proximity of the arthritic site, as it is shown that the overall serum concentration of pro-inflammatory cytokines is reduced by the administration of the disclosed preparations.

Thus, in another aspect the invention provides a method of reducing the serum concentration of pro-inflammatory cytokines e.g. in the course of inflammatory and/or autoimmune disorders such as rheumatoid arthritis, comprising the systemic administration of a cationic liposomal preparation comprising at least one active agent, and having a positive zeta potential. Preferably, the pro-inflammatory cytokines are IL-6 and/or IL-8.

The therapeutic effect of the systemic administration of a liposomal preparation having a positive zeta potential, and comprising a small molecule as active agent shown in a variety of animal models disclosed herein has not been known in the field until present.

Among other factors, the therapeutic efficacy of a composition comprising cationic liposomes and an active agent in rheumatoid arthritis depends on the binding kinetics of the liposomes to the vascular endothelial cells of the inflamed joints, the release kinetics of the drug from the liposomes, the clearance of the liposomes by liver and kidney and the clearance/release of the active agent from the site of action. So far, it could not be predicted that the different parameters are in a ratio which results in a therapeutically effective drug concentration in the respective tissue in the case of RA.

The intra-articular administration of liposomes comprising a low amount of the positively charged molecule stearylamine, and lactoferrin as an active agent has been evaluated in a mouse arthritis model {Trif, 2001 #14}, but no conclusion can be deduced regarding the systemic application of liposomes in the treatment of rheumatoid arthritis. In a more recent publication superoxide dismutase encapsulated in liposomes comprising stearylamine or pegylated liposomes comprising only uncharged lipids was evaluated {Cruz, 2005 #15}. Its was concluded that PEG-liposomes which did not comprise charged lipids had a superior therapeutic activity. Non of the two studies disclosed the feature of a positive zeta potential.

In another aspect the invention provides a method of treatment comprising the administration of a cationic liposomal preparation comprising at least one active agent, e.g. a cytotoxic or cytostatic agent in a dose which does not substantially inhibit the proliferation of endothelial cells for the prevention and/or treatment of rheumatoid arthritis or other disorders as indicated above.

In still another aspect the invention provides a method of treatment comprising the administration of a cationic liposomal preparation comprising at least one active agent, e.g. a cytotoxic or cytostatic agent in a dose which does not substantially induce apoptosis of endothelial cells for the prevention and/or treatment of rheumatoid arthritis or other disorders as indicated above. Also provided is the manufacture of a pharmaceutical composition comprising the above features. Preferably, the cytotoxic or cytostatic agent of the embodiment is a taxane, more preferably paclitaxel. In another preferred embodiment the cytostatic or cytotoxic agent is the disease-modifying anti-rheumatic drug methotrexate.

It is another aspect of the present invention to provide the use of a cationic liposomal preparation comprising no further active agent for the manufacture of a pharmaceutical composition for the prevention and/or treatment of rheumatoid arthritis or other disorders as indicated above. Within this embodiment of the invention, the active agent is the cationic liposome itself, which is not loaded with an agent having a known pharmacological effect in the prevention and/or treatment of RA or other disorders as indicated above.

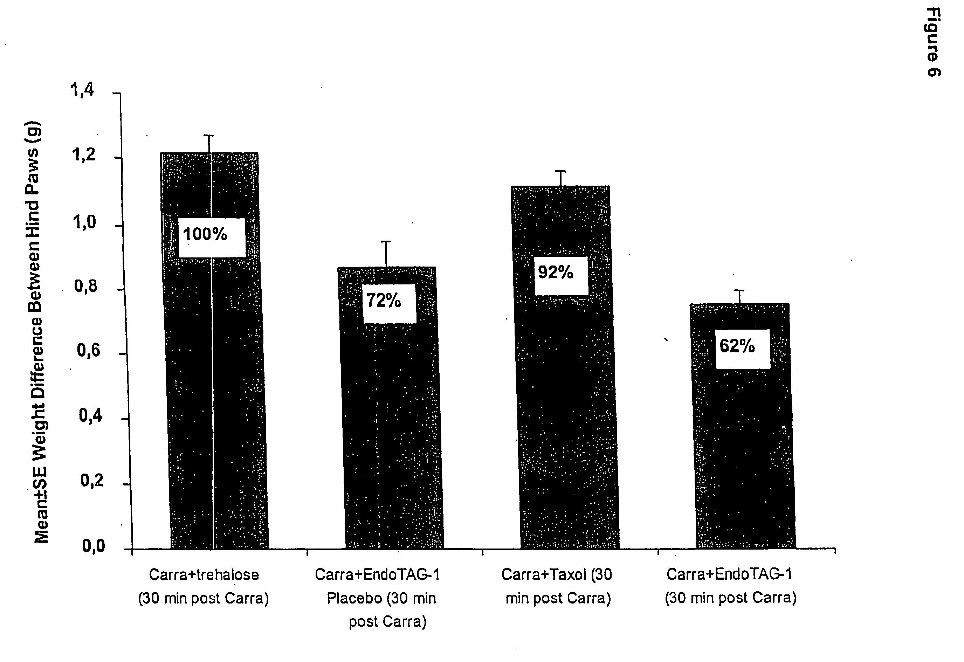

It was surprisingly found, that unloaded cationic liposomes inhibit the release of the pro-inflammatory cytokines IL-6 and IL-8 in human vascular endothelial cells stimulated by TNF-alpha in an *in vitro* model system of RA. This inhibitory effect an inflammatory processes was confirmed in several animal models of RA (Fig. 6- 10). It could also be shown that the inhibitory effect of unloaded cationic liposomes on the inflammatory process increases with the amount of cationic lipids comprised in the liposomes. Thus, the inhibitory effect of the respective liposomes increased with a positive zeta potential.

The basic principle of the present invention is not only limited to a therapeutic application within the field of rheumatoid arthritis or other disorders as indicated above but can also be utilized for diagnostic purposes. Thus, it is another aspect of the invention to use a liposomal preparation having a positive zeta potential and comprising at least one diagnostically active agent for the manufacture of a diagnostic composition for the diagnosis of rheumatoid arthritis or other disorders as indicated above by systemic administration.

In a further aspect the invention provides a composition comprising a cationic liposomal preparation comprising methotrexate. Preferably, the cationic liposomal preparation comprises a cationic lipid in an amount of at least about 40 mol%, preferably at least about 70 mol% and optionally at least one further amphiphile in an amount of up to about 50 mol% and methotrexate in an amount of up to about 20 mol%.

It has been found that methotrexate loaded liposomes comprising a very high amount of cationic lipid are stable. It has also been found that liposomal preparations comprising cationic lipids and the anionic methotrexate are stable as long as a negative net charge of the system is maintained. Upon charge neutralization of the system, precipitation occurs. It has been surprisingly found, that such liposomal preparations are stable also under conditions of charge neutralization of the system, when the system comprises small amounts of a pegylated lipid. Preferably, the liposomes comprise methotrexate in its salt, most preferably the disodium salt form.

It has been surprisingly found, that such cationic liposomes comprising methotrexate exhibit increased targeting to the proliferating vascular endothelium of tumors in mice when compared to the respcective cationic liposomes comprising no methotrexate but having the same lipid composition.

Suitable lipids for stable cationic liposomes include DOTAP, DSTAP and DPTAP. Up to date, methotrexate loaded liposomes only comprising relatively low amounts, usually less than 20 mol%, of cationic lipids were known {Patel, 1984 #16} {Pignatello, 2003 #17 {Kim, 1995 #18). Most of these liposomes comprise stearylamine as cationic agent. To obtain liposomes comprising a high load of negatively charged drugs like methotrexate that still retain a high positive zeta potential, the liposomes need to comprise a high amount of cationic lipid, but nevertheless possess a suitable stability for the respective application. This problem of known liposomal methotrexate formulations is diminished by the disclosed liposomal compositions.

The present invention provides new means for the treatment of rheumatoid arthritis with advantageous properties:
- Drugs for the treatment of RA or other disorders as indicated above can be delivered in a targeted mechanism.
- The therapeutic effect of drugs can be improved in comparison to their side effects. Several drugs used in the treatment of RA or other disorders as indicated above target basic mechanisms of the immune system and basic cellular mechanisms. In a specific embodiment the anti-inflammatory effect of methotrexate can be utilized by the systemic application of the drug with less side effects like general immunosuppression or long term toxicity which is known for MTX. Consequently, patients can stay with the treatment of a certain DMARD for a longer period and benefit from the treatment.

- A more localized action of the drugs in RA or other disorders as indicated above is achieved without the administration by an intraarticular injection, which poses the risk of an infection of the joint.
- The use of unloaded cationic liposomes offers an additional treatment option without the use of further cytotoxic or cytostatic agents.
- Biological agents, that are rather cost intensive in their production can be avoided or applied in lower amounts.
- Drugs for the treatment of RA might be administered in a lower accumulated dose over the period of treatment. This decrease of the accumulated dose might be achieved by a lowering of the single does of the drug which is administered and/or by a less frequent administration of the drug when comprised in a cationic liposome.

Detailed embodiments of the invention related to the disclosed cationic liposomal preparations, pharmaceutical compositions and other aspects of the invention related to the prevention and/or treatment and diagnosis and/or monitoring of rheumatoid arthritis or other disorders as indicated above are described in the following specification and examples.

"About" in the context of amount values refers to an average deviation of maximum +/-20 %, preferably +/-10 % based on the indicated value. For example, an amount of about 30 mol% cationic lipid refers to 30 mol% +/-6 mol% and preferably 30 mol% +/-3 mol% cationic lipid with respect to the total lipid/amphiphile molarity.

"Active agent" or "active compound" refers to an agent or compound that is diagnostically or therapeutically effective.

"Amphiphile" refers to a molecule, which consists of a water-soluble (hydrophilic) and an oil-soluble (lipophilic) part. The lipophilic part preferably contains at least one alkyl chain having at least 10, preferably at least 12 carbon atoms.

"Antifolate" refers to a drug that inhibits the activity of a folate, especially dihydrofolate, binding enzymes. An example for said enzymes is dihydrofolate reductase (DHFR).

"Carrier" refers to a diluent, adjuvant, excipient, or vehicle which is suitable for administering a diagnostic or therapeutic agent. The term also refers to a pharmaceutical acceptable component(s) that contains complexes or is otherwise associated with an agent to facilitate the transport of such an agent to its intended target site. Carriers include those known in the art, such as liposomes, polymers, lipid complexes, serum albumin, antibodies, cyclodextrins and dextrans, chelate, or other supramolecular assemblies.

"Cationic" refers to an agent that has a net positive charge or a positive zeta potential in the respective environmental condition. In the present invention, it is referred to environments where the pH is in the range between 3 and 9, preferably between 5 and 8.

"Cationic amphiphile" or "cationic lipid" refers to encompass any amphiphile or lipid which has a positive charge in the respective environmental condition. In the present invention, it is referred to environments where the pH is in the range between 3 and 9, preferably between 5 and 8.

"Cationic liposome" refers to a liposome which is positively charged in the respective environmental condition. In the present invention, it is referred to environments where the pH is in the cationic lipids or amphiphiles themselves or in admixture with other amphiphiles, particularly neutral or anionic lipids.

"Cryoprotectant" refers to a substance that helps to protect a species from the effect of freezing.

"Derivative" refers to a compound derived from some other compound while maintaining its general structural features. Derivatives may be obtained for example by chemical functionalization or derivatization.

"Drug" as used herein refers to a pharmaceutically acceptable pharmacologically active substance, physiologically active substances and/or substances for diagnosis use.

"Diagnostic or imaging label" refers to a pharmaceutical acceptable agent that can be used to localize or visualize a target region by various methods of detection. Diagnostic or imaging agents include those known in the art, such as dyes, fluorescent dyes, gold particles, iron oxide particles and other contrast agents including paramagnetic molecules, X-ray attenuating compounds (for CT and X-ray) contrast agents for ultrasound, magnetic resonance imaging (MRI) X-ray emitting isotopes (scintigraphy), and positron-emitting isotopes (PET).

"Disease modifying anti-rheumatic drug" or "DMARD" means compounds that relieve the symptoms of and help control chronic inflammatory diseases or conditions by modifying the actual disease process.

"Dose that does not significantly induce apoptosis of endothelial cells" as used herein refers to the dose of an active agent, that does not significantly induce apoptosis of endothelial cells of an inflamed joint when the cells of a subject treated with said dose are compared to the cells of an untreated subject. A significant induction of apoptosis refers to an increase of apoptosis of greater than about 50%. Apoptosis of endothelial cells can be determined by immunohistology of arthritic joint tissue. Apoptosis is represented by the ratio of cells that can be positively labelled for the endothelial cell marker CD31 to the cells that can additionally be labelled for the apoptosis marker p53. An increase of the cells that can additionally be labelled for the apoptosis marker p53 represents an induction of apoptosis. An experimental procedure for this determination is outlined in Example 9.

"Dose that does not significantly inhibit proliferation of endothelial cells" as used herein refers to the dose of an active agent, that does not significantly inhibits proliferation of endothelial cells of an inflamed joint when the cells of a subject treated with said dose are compared to the cells of an untreated subject. A significant inhibition of proliferation refers to an inhibition of greater than about 50%. Proliferation of endothelial cells can be determined by immunohistology of arthritic joint tissue. Proliferation is represented by the ratio of cells that can be positively labelled for the endothelial cell marker CD31 to the cells that can additionally be labelled for the proliferation marker PCNA or Ki67. A reduction of the cells that can additionally be labelled for the proliferation marker PCNA or Ki67 represents an inhibition of proliferation. An experimental procedure for this determination is outlined in Example 9.

"Lipid" refers to its conventional sense as a generic term encompassing fats, lipids, alcohol-ethersoluble constitutents of protoplasm, which are insoluble in water. Lipids compose the fats, fatty oils, essential oils, waxes, steroid, sterols, phospholipids, glycolipids, sulpholipids, aminolipids, chromolipids, and fatty acids. The term encompasses both naturally occurring and synthetic lipids. Preferred lipids in connection with the present invention are: steroids and sterol, particularly cholesterol, phospholipids, including phosphatidyl and phosphatidylcholines and phosphatidylethanolamines, and sphingomyelins. Where there are fatty acids, they could be about 12-24 carbon chains in length, containing up to 6 double bonds, and linked to the backbone, the fatty acids could be different (asymmetric), or there may be only 1 fatty acid chain present, e. g., lysolecithins. Mixed formulations are also possible, particularly when the non-cationic lipids are derived from natural sources, such as lecithins (phosphatidylcholines) purified from egg yolk, bovine heart, brain, or liver, or soybean.

"Liposome" refers to a microscopic spherical membrane-enclosed vesicle (about 50-2000 nm diameter) made artificially in the laboratory. The term "liposome" encompasses any compartment enclosed by a lipid bilayer. Liposomes are also referred to as lipid vesicles. In order to form a liposome the lipid molecules comprise elongated nonpolar (hydrophobic) portions and polar (hydrophilic) portions. The hydrophobic and hydrophilic portions of the molecule are preferably positioned at two ends of an elongated molecular structure. When such lipids are dispersed in water they spontaneously form bilayer membranes referred to as lamellae. The lamellae are composed of two mono layer sheets of lipid molecules with their non-polar (hydrophobic) surfaces facing each other and their polar (hydrophilic) surfaces facing the aqueous medium. The membranes formed by the lipids enclose a portion of the aqueous phase in a manner similar to that of a cell membrane enclosing the contents of a cell. Thus, the bilayer of a liposome has similarities to a cell membrane without the protein components present in a cell membrane. As used in connection with the present invention, the term liposome includes multilamellar liposomes, which generally have a diameter in the range of about 1 to about 10 micrometers and are comprised of anywhere from two to hundreds of concentric lipid bilayer alternating with layers of an aqueous phase, and also includes unilamellar vesicles which are comprised of a single lipid bilayer. The latter can be produced by subjecting multilamellar liposomes to ultrasound, by extrusion under pressure through membranes having pores of defined size, or by high pressure homogenization. A further result of these procedures is, that often well defined size distributions of the liposomes are achieved. By extrusion through membranes of defined pore size (typical values are 100, 200, 400 or 800 nm) liposomes with a size distribution close to the pore size of the membrane can be achieved. By ultrasound and high pressure homogenisation procedures, defined size distributions are obtained by molecular self-organization as a function of the experimental conditions.

"Liposomal preparation" and "liposomes" are used synonymously throughout the present application. The liposomal preparation may be a component of a "pharmaceutical composition" and may be administered together with physiologically acceptable excipients such as a buffer.

"Lysolipid" refers to a lipid where one fatty acid ester has been cleaved resulting in a glycerol backbone bearing one free hydroxyl group.

"Lysophospholipid" refers to a phospholipid where one fatty acid ester has been cleaved resulting in a glycerol backbone bearing one free hydroxyl group.

"Methotrexate" (MTX) refers to the drug with the chemical names N-[4-[[(2,4-diamino-6-pteridinyl)methyl]methylamino]benzoyl]-L-glutamic acid or (2S)-2-[[4-[[(2,4-diaminopteridin-6 yl)methyl]methylamino]benzoyl]amino] pentanedioic acid that is also known under the trivial name 4-amino-10-methylfolic acid. The molecular formula of MTX is C₂₀H₂₂N₈O₅. As used herein, methotrexate also refers to pharmaceutically acceptable derivatives, e.g. salts, esters or amides of methotrexate. Pharmaceutically acceptable salts can be selected from, but are not limited to, alkali metal salts such as sodium or potassium, alkaline earth salts or an ammonium salt (all of which are herein referred to as a pharmaceutically acceptable salts). In some embodiments, methotrexate refers to methotrexate disodium. Methotrexate also refers to acetylated forms, benzhydryl-sulfinylacetic acid forms, sulfone forms, hydroxylated forms, polymorphic forms, analogs, derivatives, cogeners, prodrugs, metabolic acids and compounds made by mixtures thereof. The methotrexate of the present invention also includes individual enantiomers or racemic mixtures of methotrexate.

"Mol percent" or "mol%" defines a fraction of a concentration. Mol percent e.g. refers to the ration (given in percent) of moles of a lipid or a lipid-soluble species to its total lipid content (given in moles) in 1 liter. For example, one liter of a liposomal formulation comprising 5 mmol of DOTAP, 4.7 mmol of DOPC, and 0.3 mmol of paclitaxel is 10 mM with respect to its total lipid content. In this case, DOTAP contributes 50 mol percent to the liposomal formulation, DOPC 47 and paclitaxel 3 mol percent. This formulation can be described as 10 mM DOTAP/DOPC/Paclitaxel 50/47/3.

"Negatively charged lipids" refer to lipids that have a negative net charge in the respective environmental condition.

"Rheumatoid arthritis" refers to an autoimmune disease which causes chronic inflammation of the joints, the tissue around the joints, as well as other organs in the body.

"Taxane" as used herein refers to the class of antineoplastic agents having a mechanism of microtubule action and having a structure that includes the unusual taxane ring structure and a stereospecific side chain that is required for cytostatic activity. Also included within the term "taxane" are a variety of known derivatives, including both hydrophilic derivatives, and hydrophobic derivatives. Taxane derivatives include, but not limited to, galactose and mannose derivatives described in International Patent Application No. WO99/18113; piperazino and other derivatives described in WO 99/14209; taxane derivatives described in WO 99/09021, WO 98/22451, and U. S. Patent No. 5,869, 680; 6-thio derivatives described in WO 98/28288; sulfenamide derivatives described in U. S. Patent No. 5,821, 263; and taxol derivative described in U. S. Patent No. 5,415, 869. "Paclitaxel" (which should be understood herein to include analogues, formulations, and derivatives such as, for example, docetaxel, taxotere (a formulation of docetaxel), 10-desacetyl analogs of paclitaxel and 3'N-desbenzoyl-3'N-t-butoxycarbonyl analogs of paclitaxel) may be readily prepared utilizing techniques known to those skilled in the art (see also WO 94/07882, WO 94/07881, WO 94/07880, WO 94/07876, WO 93/23555, WO 93/10076; U. S. Pat. Nos. 5,294, 637; 5,283, 253; 5,279, 949; 5,274, 137; 5,202, 448; 5,200, 534; 5,229, 529; and EP 590,267), or obtained from a variety of commercial sources, including for example, Sigma Chemical Co. , St. Louis, Mo. (T7402 from Taxus brevifolia ; or T-1912 from Taxus yannanensis). Paclitaxel should be understood to refer to not only the common chemically available form of paclitaxel, but analogs (e. g. , taxotere, as noted above) and paclitaxel conjugates (e.g., paclitaxel-PEG, paclitaxeldextran, or paclitaxel-xylose).

"Particle diameter" refers to the size of a particle. To experimentally determine particle diameters, dynamic light scattering (DLS) measurements, using Malvern Zetasizer 1000 or 3000 (Malvern, Herrenberg, Germany) were performed. For quantitative data analysis (determination of Z (average and PI)), in all cases parameters (refractive index, density, viscosity) of pure water were plugged in, even if the aqueous phase contained cryoprotectants to a certain extend. Therefore, for the absolute numbers, a certain systematic deviation with respect to literature data may have to be taken into account.

"Pegylated lipid" refers to a lipid bearing one ore more polyethylene glycol residues.

"Pharmaceutical composition" refers to a combination of two or more different materials with superior pharmaceutical properties than are possessed by either component.

"Phospholipid" refers to a lipid consisting of a glycerol backbone, a phosphate group and one or more fatty acids wich are bound to the glycerol backbone by ester bonds.

"Positively charged lipids" refer to a synonym for cationic lipids (for definition see definition of "cationic lipids"). In the present invention, it is referred to environEPments where the pH is in the range between 3 and 9, preferably between 5 and 8.

"Sterol" refers to a steroid alcohol. Steroids are derived from the compound called cyclopentanoperhydrophenanthrene. Well-known examples of sterols include cholesterol, lanosterol, and phytosterol.

"Therapeutic agent" refers to a species that reduces the extent of the pathology of a disease such as rheumatoid arthritis or other disorders as indicated above.

"Treatment", "treating", "treat" and the like are used herein to generally mean obtaining a desired pharmacologic and/or physiologic effect. The effect may be prophylactic in terms of completely or partially preventing a disease or symptom thereof and/or may be therapeutic in terms of a partial or complete stabilization or cure for a disease and/or adverse effect attributable to the disease. "Treatment" as used herein covers any treatment of a disease in a mammal, particularly a human, and includes: (a) preventing the disease or symptom from occurring in a subject which may be predisposed to the disease or symptom but has not yet been diagnosed as having it; (b) inhibiting the disease symptom, i.e., arresting its development; or (c) relieving the disease symptom, i.e., causing regression of the disease or symptom.

"Zeta potential" refers to measured electrical potential of a colloidal particle in aqueous environment, measured with an instrument such as a Zetasizer 3000 using Laser Doppler micro-electrophoresis under the conditions specificed. The zeta potential describes the potential at the boundary between bulk solution and the region of hydrodynamic shear or diffuse layer. The term is synonymous with "electrokinetic potential" because it is the potential of the particles which acts outwardly and is responsible for the particle's electrokinetic behaviour.

The active agent comprised in the disclosed liposomal preparation can be a disease-modifying anti-rheumatic drug or a non-steroidal anti-inflammatory drug. The group of DMARDs comprises small molecule agents like doxycycline, chondroitin sulfate, the antifolate methotrexate, leflunomide, sulphasalazine, penicillamine, gold salts like aurothiomalate, cyclophosphamide, azathioprine, cyclosporine A, minocycline, glucocorticoids and antimalaria drugs as well as pharmacologically active derivatives of any of the molecules. A preferred small molecule DMARD is methotrexate.

The group of DMARDs also comprises biological DMARDs like TNF-alpha antagonists such as Etanercept (Enbrel®, Amgen), adalimumab (Humira® Abott), infliximab (Remicade®, Centocor), CDP870 or IL-1 receptor antagonists such as the Interleukin-1 receptor antagonist Kineret®, or RGD peptides. A preferred embodiment of biological DMARDs are RGD peptides. The peptides can be linear or cyclic, potentially the peptides are derivatized. The DMARD may also be an inhibitor of matrix metalloproteinases (MMPs), especially an inhibitor of MMP-1, MMP-2, MMP-3, MMP-7, MMP-8, MMP-9, MMP-10, MMP-11, MMP-13 or TNF-alpha-converting enzyme (TACE).

The group of non-steroidal drugs comprises enolic acids such as piroxicam, tenoxicam and meloxicam, heteroaryl acetic acids such as diclofenac, tolmetin, ketorolac, misoprostol and zomepirac; indole and indene acetic acids such as indomethacin, mefenamic acid, sulindac and etodolac; para-amino phenol derivates such as phenacetin and acetaminophen; propionic acids including naproxen, flurbiprofen, fenoprofen, oxaprozin, carprofen, ketoprofen and ibuprofen; fenamates including mefenamic acid, meclofenamate and flufenamic acid; alkanones such as nabumetome; pyrazolones including phenylbutazone, oxyphenbutazone, antipyrine, aminopyrine and kebuzone, salicylates including acetyl salicylate (aspirin), salicylate, salsalate, difunisal, olsalazine, fendosal, sulfasalazine and thiosalicylate, COX-2 inhibitors such as celecoxib (CELEBREX®), valdecoxib (EXTRA®), etoricoxib (ARCOXIA®), lumiracoxib (PREXIGE®), parecoxib, and rofecoxib (VIOXX®), deracoxib (DERAMAXX®) and methylfulfonyl compounds. In a preferred embodiment the non-steroidal drug is selected from COX-2 inhibitors.

In another preferred embodiment, the active agent is selected from a cytotoxic or cytostatic agent, preferably an antineoplastic agent especially antimitotic agent like a taxane, an anthracyclin preferably doxorubicin or epirubicin, a statin, a depsipeptide, thalidomide, other agents interacting with microtubuli such as discodermolide, laulimalide, isolaulimalide, eleutherobin, Sarcodictyin A and B, antimetabolites preferably antifolates, alkylating agents especially platinum containing compounds like cisplatin, carboplatin, DNA topoisomerase inhibiting agents like camptothecin, RNA/DNA antimetabolites, especially 5-fluorouracil, gemcitabine or capecitabine. In a more preferred embodiment, it is selected from paclitaxel, docetaxel, camptothecin or any derivative thereof.

In a preferred embodiment of the present invention, the taxane is paclitaxel or a derivative thereof. The cationic liposomal preparation may comprise paclitaxel in an amount of at least about 2 mol% to about 8 mol%, preferably from at least 2.5 mol% to about 3.5 mol%. The therapeutic application of paclitaxel is shown in Fig. 6-9.

It is preferred that the antifolate is methotrexate.

In a further preferred embodiment the cationic liposomal preparation comprises DOTAP, DOPC and paclitaxel in a ratio of about 50:47:3. This formulation is also designated MBT-0206 or EndoTAG-1. EndoTAG-1 has a lipid content of 10 mM in a 10% m/m trehalose dihydrate solution. The manufacture of such a formulation is disclosed in WO 2004/002468.

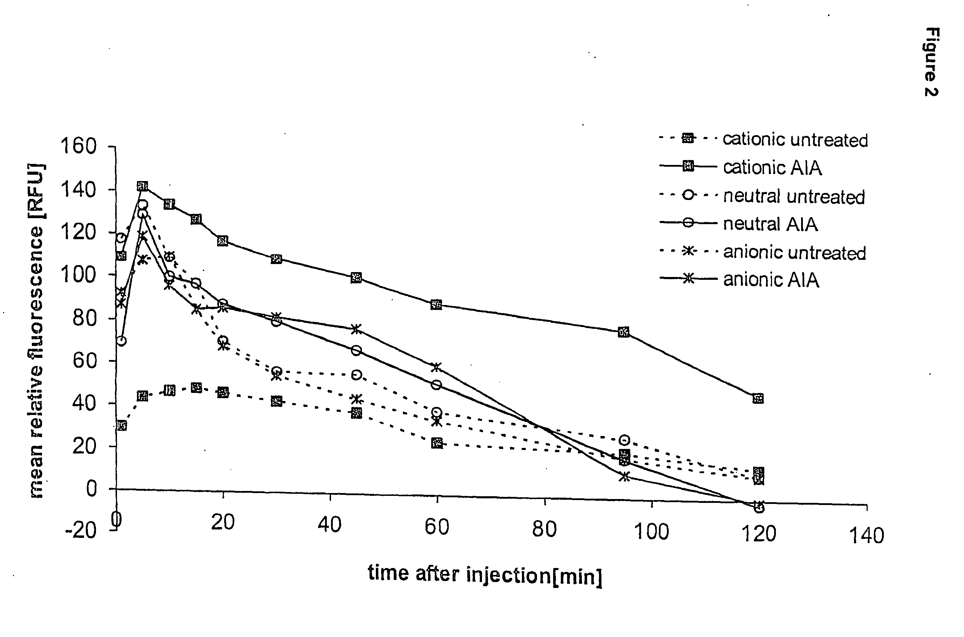

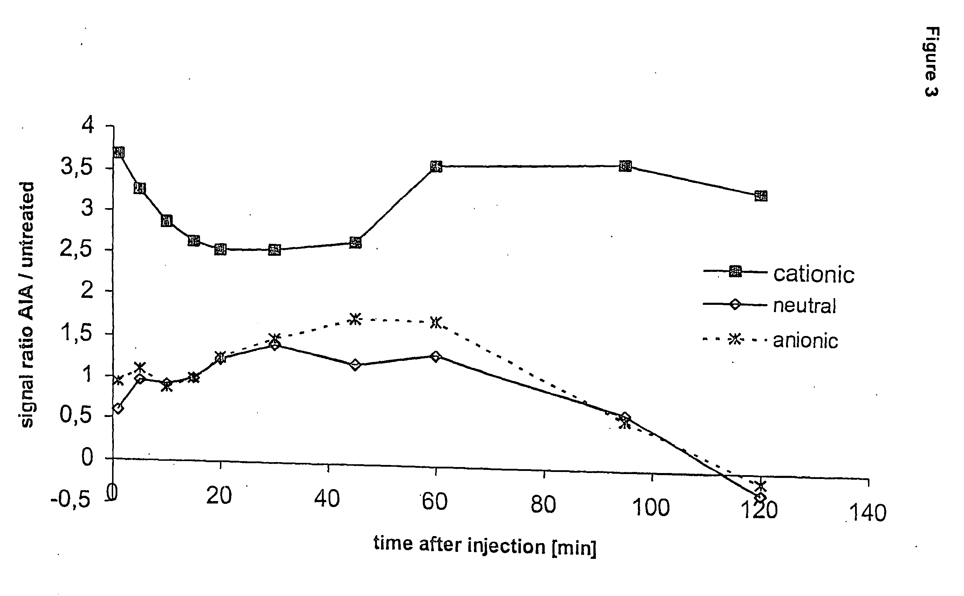

Fig 2 and 3 of the present application depict the targeting of synovial vessels of arthritic mice or control mice by neutral, positive and negative rhodamine labelled liposomes. For the cationic liposomes, a comparably high ratio between the signal of labelled synovial cells in untreated versus treated (arthritic) mice can be observed, indicating a specific targeting of cationic liposomes to the vascular endothelium in arthritic mice.

Thus, the present invention provides a method for the selective delivery of at least one active agent to the vascular endothelium of arthritic joints, comprising the systemic administration of a cationic liposomal preparation having a positive zeta potential. The endothelial cell layer constitutes the barrier between the blood and the synovial tissue. Hence, it is another embodiment of the present invention to provide a method for the selective delivery of at least one active agent to the synovial tissue of arthritic joints, comprising the systemic administration of a cationic liposomal preparation having a positive zeta potential.

It is a preferred embodiment of the present invention that the liposomal preparation can be administered systemically, preferably intravenously, more preferably by infusion or injection. When administered by infusion over a longer period of time, the rate of infusion may be increase over time. If possible, a small volume is used for administration. The compositions of the present invention may be administered alone or in a combination therapy with another agent for the treatment of RA or other disorders as indicated above.

It is one aspect of the invention to use the provided compositions for the administration in a low dose. Within this context, a low dose refers to the dose of an active agent which is administered in form of the cationic liposomal preparation. This low dose is characterized by being lower than the dose of the active agent, e.g. a cytostatic or cytotoxic agent, preferably methotrexate, that is administered in the clinical treatment of RA or the treatment of cancer to date.

In a preferred embodiment of the invention, the liposomal preparation or the pharmaceutical composition comprising said preparation is administered in a dose that does not substantially inhibit the proliferation or substantially induce apoptosis of endothelial cells. Preferably, the dose does not substantially inhibit the proliferation or substantially induce apoptosis of endothelial cells of a human patient after administration of the inventive composition/preparation to said human patient.

In another preferred embodiment the dose does not substantially inhibit the proliferation and/or substantially induce apoptosis of endothelial cells of a mammal, when administered to a mammal, e.g. a human patient. The person skilled in the art is able to determine said dose. A possible experimental set-up is disclosed in the present application, wherein the suitable dose is determined in an experimental animal. The dose can also be determined by alternative means that are suitable for determining the proliferation or induction of apoptosis of endothelial cells in the human body or the body of a mammal. The experimental animal may be a rodent, e.g. a mouse, a rat, a hamster, a rabbit or a guinea pig, or a dog.

In another aspect a cationic liposomal preparation for systemic administration having a positive zeta potential and comprising at least one diagnostic or imaging label as an active agent is provided. This preparation can be utilized for diagnosis and/or monitoring of RA or other disorders as indicated above or other diagnostic purposes within the field of RA or other disorders as indicated above. The diagnostic or imaging label may be selected from a group comprising fluorescent labels, histochemical labels, immunohistochemical labels, radioactive labels, especially metal ions or metal ion chelates (perferably chelates from transition metals such as gadolinium, lutetium, or europium) for example as used for MRI, CT and X-ray contrast. Other preferred labels are radioisotopes, preferably isotopes of Iodine, Indium Gallium, Ruthenium, Mercury, Rhenium, Tellurium, Thulium, and more preferably Technetium.

In a preferred embodiment, the liposomal preparation comprises a cationic lipid or a mixture of cationic lipids in an amount of at least about 30 mol%, more preferably at least about 50 mol%, and most preferably at least about 80 mol%.

The preferred cationic lipids of the liposomal preparation are N-[1-(2,3-dioleoyloxy)propyl]-N,N,N-trimethyl ammonium (TAP) salts, e.g. the methylsulfate. Preferred representatives of the family of TAP lipids are DOTAP (dioleoyl-), DMTAP (dimyristoyl-), DPTAP (dipalmitoyl-), or DSTAP (distearoyl-). Other useful lipids for the present invention may include: DDAB, dimethyldioctadecyl ammonium bromide; 1,2-diacyloxy-3-trimethylammonium propanes, (including but not limited to: dioleoyl, dimyristoyl, dilauroyl, dipalmitoyl and distearoyl; also two different acyl chain can be linked to the glycerol backbone); N-[1-(2,3-dioleyloxy)propyl]-N,N-dimethyl amine (DODAP); 1,2-diacyloxy-3-dimethylammonium propanes, (including but not limited to: dioleoyl, dimyristoyl, dilauroyl, dipalmitoyl and distearoyl; also two different acyl chain can be linked to the glycerol backbone); N-[1-(2,3-dioieyloxy)propyl]-N,N,N-trimethylammonium chloride (DOTMA); 1,2-dialkyloxy-3-dimethylammonium propanes, (including but not limited to: dioleyl, dimyristyl, dilauryl, dipalmityl and distearyl; also two different alkyl chain can be linked to the glycerol backbone); dioctadecylamidoglycylspermine (DOGS); 3β-[N-(N',N'-dimethylaminoethane)carbamoyl]cholesterol (DC-Chol); 2,3-dioleoyloxy-N-(2-(sperminecarboxamido)-ethyl)-N,N-dimethyl-1-propanaminium trifluoroacetate (DOSPA); β-alanyl cholesterol; cetyl trimethyl ammonium bromide (CTAB); diC14-amidine; N-*tert*-butyl-N'-tetradecyl-3-tetradecylaminopropionamidine; 14Dea2; N-(alpha-trimethylammonioacetyl)didodecyl-D-glutamate chloride (TMAG); O,O'-ditetradecanoyl-N-(trimethylammonioacetyl)diethanolamine chloride; 1,3-dioleoyloxy-2-(6-carboxy-spermyl)-propylamide (DOSPER); N,N,N',N'-tetramethyl-N,N'-bis(2-hydroxylethyl)-2,3-dioleoyloxy-1,4-butanediammonium iodide; 1-[2-(acyloxy)ethyl]2-alkyl (alkenyl)-3-(2-hydroxyethyl)-imidazolinium chloride derivatives as described by Solodin et al. (Solodin et al., 1995), such as 1-[2-(9(Z)-octadecenoyloxy) ethyl]-2-(8(Z)-heptadecenyl-3-(2-hydroxyethyl)imidazolinium chloride (DOTIM), 1-[2-(hexadecanoyloxy)ethyl]-2-pentadecyl-3-(2-hydroxyethyl) imidazolinium chloride (DPTIM), 2,3-dialkyloxypropyl quaternary ammonium compound derivatives, containing a hydroxyalkyl moiety on the quaternary amine, as described e.g. by Felgner et al. (Felgner et al., 1994) such as: 1,2-dioleoyl-3-dimethyl-hydroxyethyl ammonium bromide (DORI), 1,2-dioleyloxypropyl-3-dimethyl-hydroxyethyl ammonium bromide (DORIE), 1,2-dioleyloxypropyl-3-dimetyl-hydroxypropyl ammonium bromide (DORIE-HP), 1,2-dioleyloxypropyl-3-dimethyl-hydroxybutyl ammonium bromide (DORIE-HB), 1,2-dioleyloxypropyl-3-dimethyl-hydroxypentyl ammonium bromide (DORIE-Hpe), 1,2-dimyristyloxypropyl-3-dimethyl-hydroxylethyl ammonium bromide (DMRIE), 1,2-dipalmityloxypropyl-3-dimethyl-hydroxyethyl ammonium bromide (DPRIE), 1,2-disteryloxypropyl-3-dimethyl-hydroxyethyl ammonium bromide (DSRIE); cationic esters of acyl carnitines as reported by Santaniello et al. [US5498633]; cationic triesters of phosphatidylcholine, i.e. 1,2-diacyl-sn-glycerol-3-ethylphosphocholines. The hydrocarbon chains of the cationic lipids can be saturated or unsaturated and branched or non-branched with a chain length from C₁₂ to C₂₄. Preferably, the lipid comprises two hydrocarbon chains which may be different or identical.

The liposomal preparation optionally comprises at least one neutral and/or anionic lipid. Neutral lipids are lipids which have a neutral net charge. Anionic lipids or amphiphiles are molecules which have a negative net charge. These can be selected from sterols or lipids such as cholesterol, phospholipids, lysolipids, lysophospholipids, sphingolipids or pegylated lipids with a neutral or negative net change. Useful neutral and anionic lipids thereby include: phosphatidylserine, phosphatidylglycerol, phosphatidylinositol (not limited to a specific sugar), fatty acids, sterols, containing a carboxylic acid group for example, cholesterol, 1,2-diacyl-sn-glycero-3-phosphoethanolamines (PE), including, but not limited to 1,2-dioleoyl-sn-glycero-3-phosphoethanolamine, (DOPE), or 1,2-distearoyl-sn-glycero-3-phosphoethanolamine (DSPE), 1,2-diacyl-glycero-3-phosphocholines, including, but not limited to 1,2-dioleoyl-sn-glycero-3-phosphocholine (DOPC), or 1,2-distearoyl-sn-glycero-3-phosphocholine (DSPC) and sphingomyelin. The fatty acids linked to the glycerol backbone are not limited to a specific length or number of double bonds. Phospholipids may also have two different fatty acids. Preferably, the further lipids are in the liquid crystalline state at room temperature and they are miscible (i.e. a uniform phase can be formed and no phase separation or domain formation occurs) with the used cationic lipid, in the ratio as they are applied. In a preferred embodiment the neutral and/or anionic lipids are DOPC and/or DOPE. The liposomal preparation may comprise neutral and/or anionic lipids, preferably DOPC and/or DOPE in an amount of up to about 70 mol%, preferably up to about 30 mol% and more preferably up to about 10 mol%.

In a further preferred embodiment of the present invention, a composition comprising a cationic liposomal preparation comprising a cationic lipid or a mixture of cationic lipids in an amount of at least about 30 mol% and methotrexate is provided. More preferably the composition comprises at least about 50 mol%, and most preferably at least about 80 mol% cationic lipid. Preferably, the composition comprises methotrexate in an amount of up to 20 mol%, e.g. in an amount of 5 to 20 mol%. Optionally the composition comprises at least a further neutral or negative amphiphile in an amount of up to about 70 mol%, preferably up to about 50 mol%, most preferably up to 20 mol%. Preferably, the composition comprises the lipids described above. Preferred cationic lipids are DOTAP, DSTAP and or DPTAP, more preferably DOTAP. Preferred neutral and/or anionic lipids are DOPC and/or DOPE. The lipids, e.g. the neutral and/or anionic lipids such as DOPC and/or DOPE may be pegylated. Preferably, the molar ratio between the lipids of the composition and the methotrexate is greater than 5 to 1.

Preferred embodiments are a composition comprising 90 mol% DOTAP and 10 mol% MTX and a composition comprising 85 mol% DOTAP, 5 mol% pegylated DOPE, and 10 mol% MTX.

Preferably, the sodium salt of MTX is utilized for the preparation of the preparations/compositions of the present invention.

The liposomal preparation of the invention may also comprise pegylated lipids. Pegylated lipid refers to a lipid bearing one ore more polyethylene glycol residues. The lipid bearing the polyethylene glycol residue may be an anionic or cationic and preferably a neutral lipid. In a preferred embodiment the neutral and/or anionic lipid comprises a pegylated phosphoethanolamine (PE) such as DOPE and/or DSPE, most preferably DOPE pegylated with PEG₂₀₀₀. The liposomal preparation of the invention may also comprise lipids which are derivatized by other biocompatible polymers.

The liposomal preparations of the present invention can be obtained by homogenizing the hydrophobic compounds in water by a suitable method and further processing. Homogenizing can be obtained by mechanical mixing, stirring, high-pressure homogenization, addding an organic phase comprising the hydrophobic compounds to the aqueous phase, spraying techniques, supercrtitical fluid technology or any other technique suitable in order to obtain lipid dispersions in water.

In a preferred embodiment, the liposomal preparations of the present invention can be obtained by method like the "film method" or by organic solution injection, which are known to those skilled in the art (Janoff, A. S. (ed): Liposomes, Rational Design, Marcel Dekker, New York 1999). Further methods for the production of cationic liposomal preparations are disclosed in WO 2004/002468.

The cationic liposome preparation can be dehydrated, stored for extended periods of time while dehydrated, and then rehydrated when and where it is to be used, without losing a substantial portion of its contents during the dehydration, storage and rehydration processes. To achieve the latter, one or more protective agents, such as cryoprotectants, may be present. Thus, the inventive cationic liposome preparation preferably comprises a cryoprotectant, wherein the cryoprotectant is selected from a sugar or an alcohol or a combination thereof. Preferably, the cryoprotectant is selected from trehalose, maltose, sucrose, glucose, lactose, dextran, mannitol or sorbitol.

Preferably, the liposomal preparation comprises trehalose in the range of about 5 % (m/v) to about 15 % (m/v) with respect to the total volume of the preparation.

In a preferred embodiment the liposomal preparation comprises a zeta potential of greater than about 20 mV, preferably greater than about 30 mV, and most preferably greater than about 40 mV when measured in about 0.05 mM KCI solution at about pH 7.5.

Preferred liposomes of the liposomal preparations disclosed in the present application are small unilamellar liposomes with an average particle diameter of about 50 nm to about 400 nm, preferably about 100 nm to about 300 nm, about 100 nm to about 200 nm.

In accordance with other aspects of the invention, the pharmaceutical composition of the invention comprises a pharmaceutically effective amount of the inventive liposomal preparation together with a pharmaceutically acceptable carrier, diluent and/or adjuvant is provided.

It should be noted that all preferred embodiments discussed for one or several aspects of the invention also relate to all other aspects. This particularly refers to the amount and type of cationic lipid, the amount and type of neutral and/or anionic lipid, the amount and type of active agent, the amount and type of further active agent for combination therapy, and the type of disorder to be treated.

The following examples should be illustrative only but are not meant to be limiting to the scope of the invention. Other generic and specific embodiments will be apparent to those skilled in the art.

### Figure legends:

**Fig. 1**
   *In vivo* binding kinetics of Rhodamine labelled cationic liposomes to synovial vessels of arthritic AIA (A) or healthy (B) C57Black6 mice. Shown are fluorescence images from representative areas of synovial tissues of four different animals analysed by intravital microscopy at 4 different time points.
**Fig. 2**
   Targeting of cationic, neutral and anionic Rhodamine labelled liposomes to synovial vessels of untreated/healthy or arthritic AIA C57Black6 mice was analysed by obtaining quantitative fluorescence kinetic data, using intravital microscopy. Shown is the mean relative fluorescence determined at three functional synovial vessels of 6 animals per group. Data were recorded at the 10 indicated time points over 120 min.
**Fig. 3**
   Selectivity of liposome targeting to synovial vessels of arthritic AIA C57Black6 mice was displayed as the ratio of the mean relative fluorescence signal obtained in AIA mice to the signal obtained in untreated mice (shown in Fig. 2) at the corresponding time point.
**Fig. 4**
   HUVEC were incubated either with 1, 50, 100 or 500 nM EndoTAG-1 or EndoTAG-Placebo in EGM2 full medium (5 % FBS) containing TNF-alpha (30 U/ml) when indicated. Supernatant was harvested after 48 hrs and amount of IL-6 (left graph) and IL-8 (right graph) cytokines were measured using "BD Cytometric Bead Array".
**Fig. 5**
   Targeting of cationic Rhodamine labelled liposomes to the striated skin muscle layer of Syrian golden hamsters after superfusion with NaCl or TNF-alpha was analysed by obtaining quantitative fluorescence kinetic data using intravital microscopy. Shown is the mean relative fluorescence determined in the skinfold chamber from 6 animals per group. Data were recorded at the 18 indicated time points over 360 min.
**Fig. 6**
   The therapeutic effect of EndoTAG-1 on rat Carrageenan-induced paw inflammation was tested in Sprague Dawley rats. Shown is the mean weight difference of hind paws from 6 different animals 4h after injection of the Carrageenan into the right hind footpad. Trehalose, EndoTAG-1 placebo, Taxol® or EndoTAG-1 was administered i.v. 30 min after Carrageenan injection.
**Fig. 7**
   The therapeutic effect of EndoTAG-1 on collagen induced arthritis in DBA/1 mice was measured as a decrease in the frequency of paws showing ankylosis. Groups of 15 animals were treated from day 7 with trehalose (disease control), EndoTAG1-placebo, Taxol® and EndoTAG-1. Shown is the mean frequency of paw ankylosis per animal from day 19 to day 37.
**Fig. 8**
   The therapeutic effect of EndoTAG-1 on collagen induced arthritis in B10RIII mice was measured as a decrease in mean clinical arthritis score of all paws. Groups of 15 animals were treated from the first day with a clinical sign on one of the four paws (day 0) with trehalose (disease control), EndoTAG1-placebo, Taxol® and EndoTAG-1. Shown is the mean clinical arthritis score per animal from day 0 to day 11.
**Fig. 9**
   The therapeutic effect of EndoTAG-1 on antigen induced arthritis in C57Black6 mice was measured as a decrease in swelling/diameter of the mBSA injected hind knee. Groups of 10 animals were treated from day 0 (time point of mBSA injection/disease induction) with trehalose (disease control), EndoTAG1-placebo, Taxol® and EndoTAG-1. Shown is the mean difference in the injected left hind knee diameter compared to the knee diameter before diease induction.
**Fig. 10**
   The therapeutic effect of EndoMTX on collagen induced arthritis in DBA/1 mice was measured as a decrease in the clinical arthritis score of all paws. Groups of 15 animals were treated from day 15 with trehalose (disease control), EndoMTX-placebo, MTX and EndoMTX. Shown is the mean clinical arthritis score per animal from day 14 to day 39.
**Fig. 11**
   The therapeutic effect of EndoMTX on inflammation (A), pannus formation (B) and cartilage damage (C) was measured by assessing histopathological changes (scored 0-4) in hind knee joints in DBA/1 mice developing CIA. Groups of 15 animals were treated from day 15 with Trehalose (disease control), EndoMTX-placebo, MTX and EndoMTX. Shown is the mean histological score per knee joint.
**Fig. 12**
   The therapeutic effect of EndoMTX on inflammation (A), pannus formation (B) and cartilage damage (C) was measured by assessing histopathological changes (scored 0-4) in all paws in DBA/1 mice developing CIA. Groups of 15 animals were treated from day 15 with Trehalose (disease control), EndoMTX-placebo, MTX and EndoMTX. Shown is the mean histological score per paw.
**Fig. 13**
   The therapeutic effect of EndoMTX treatment on bone destruction was measured by assessing osteoclast numbers in hind knee joints of mice developing CIA. Groups of 15 animals were treated from day 15 with Trehalose (disease control), EndoMTX-placebo, MTX and EndoMTX. Shown is the mean osteoclast number per hind knee joint.
**Fig. 14**
   **EndoMTX protects from bone resorption.**
   Sections were stained with the tartrate-resistant acid phosphatase method (TRAP) and analysed for osteoclasts and bone resorption. Black arrows indicate bone resorption. Pictures 1-4 (Trehalose group) demonstrate strong bone resorption at sites of osteoclast staining. Pictures 2 and 4 show bone resorption of compact bone of the diaphysis and pictures 1 and 3 resorption of epiphyseal bone underlying the articular surfaces. Bone from EndoMTX treated animals (pictures 5-8) do not show bone resorption of compact bone of the diaphysis or epiphyseal bone.
**Fig. 15**
   **IL-6 analysis of day 39 serum samples from DBA/1 mice developing CIA.**
   Strong suppression of IL-6 or KC serum levels of mice developing CIA after EndoMTX treatment. Groups of 15 animals were treated from day 15 with Trehalose (disease control), EndoMTX-placebo, MTX and EndoMTX. Shown is the mean protein concentration detected in serum per animal.
**Fig. 16**
   The therapeutic effect of DOATP liposomes on rat Carrageenan-induced paw inflammation was tested in Sprague Dawley rats. Shown is the mean weight difference of hind paws from 6 different animals 4h after injection of the Carrageenen into the right hind footpad. Trehalose and DOTAP/DOPC liposomes with a molar ratio 100/0, 75/25, 50/50, 25/75 and 5/95 mol% were administered iv 30 min after Carrageenen injection.
**Fig. 17**
   Targeting of cationic Rhodamine labelled DOTAP liposomes with or without MTX to normal or tumor vessels of hamsters was analysed by obtaining quantitative fluorescence kinetic data, using intravital microscopy. Shown is the mean area below fluorescence time curves of 6 animals per group.
**Fig. 18**
   The therapeutic effect of EndoTAG-1 on collagen induced arthritis in B10RIII mice was measured as a decrease in mean clinical arthritis score of all paws. Groups of 15 animals each were treated from the first day with a clinical sign on one of the four paws (day 0) with trehalose (disease control), EndoTAG1-placebo + Taxol® and EndoTAG-1. Shown is the mean clinical arthritis score per animal from day 0 to day 13.
**Fig. 19**
   The therapeutic effect of EndoMTX on collagen induced arthritis in DBA/1 mice was measured as a decrease in the clinical arthritis score of all paws. Groups of 13 animals each were treated from day 15 with trehalose (disease control), MTX and EndoMTX. Shown is the mean clinical arthritis score per animal (sum score of four paws) from day 19 to day 39.
**Fig. 20**
   The therapeutic effect of EndoTAG-1 combined with Enbrel on collagen induced arthritis in B10RIII mice was measured as a decrease in mean clinical arthritis score of all paws. Groups of 15 animals each were treated from the first day with a clinical sign on one of the four paws (day 0) with trehalose (disease control), EndoTAG-1, Enbrel and EndoTAG-1 combined with Enbrel. Shown is the mean clinical arthritis score per animal from day 0 to day 11.

### Examples

### Example 1: Targeting of cationic liposomes to synovial vessels of arthritic AIA C57Black6 mice

Female C57Black6 mice with an age of 8-10 weeks at arrival, weighing 15-17 grams, were purchased from Charles River and housed in isolated cages under save environmental conditions (5 mice per cage, 22°C, 30-70% humidity and 12h light/dark cycle) with food and water ad libitum. Experimental design was reviewed and approved by local government.

Mice were assigned to the experimental groups acclimated at least 14 days before induction of antigen-induced arthritis (AIA). Animals were anaesthetized with Isoflurane and immunized by a subcutaneous injection of 100 µg of methylated bovine serum (mBSA; Sigma, Deisenhofen Germany), dissolved in 50 µl of Complete Freund's Adjuvant (Sigma) and supplemented with Mycobacterium tuberculosis H37 RA (Difco) and an additional intraperitoneal injection of 2 · 10⁹ heat-killed Bordetella pertussis (DSMZ, Germany) on days-21 and -14 prior to induction of arthritis as described by Brackertz et al {Brackertz, 1977 #19}. Arthritis was induced on day 0 by injection of 100 µg mBSA in 50 µl saline into the left knee joint using a sterile 33-gauge microcannula. Control animals underwent the same procedure but received only equivalent volumes of saline.

Mice were anaesthetized by inhalation of isoflurane 1.5% (Forence, Abbott, Wiesbaden, Germany) and a mixture of O₂/N₂O, using a vaporizer (Dräger, Lübeck, Germany). Arterial and venous catheters were implanted into the tail. The mean arterial blood pressure was measured using the arterial catheter. Animals were kept on a heating pad to stabilize body temperature. The left hind limb was placed on a stage with the knee slightly flexed. Through a 1-cm incision distal to the patellar tendon, the tendon was carefully mobilized, cut transversally, and elevated to provide visual access to the intra-articular synovial tissue as described by Veihelmann et al. {Veihelmann, 1997 #20}.

The microscopic setup has previously been described by Haris et al. {Haris, 1997 #21}. An intravital microscope (Zeiss, Oberkochen, Germany) equipped with a 20-fold water immersion objective (total magnification 432-fold: Zeiss, Jena, Germany) was used to visualize the synovial microcirculation. Three regions of interest were selected, each containing postcapillary venules (18-40 µm in diameter) or arterioles (8-14 µm in diameter), as well as capillaries for the measurement of functional capillary density (FCD). By means of a computer-controlled platform which moves step by step driven by the computer, identical vessel segments were reinvestigated with respect to the parameters described above. For visualization of plasma and blood vessels, a variable 12-volt, 100-watt halogen light source and the Zeiss filter set 09 [band pass (BP) 450-490, dichroic filter (FT) 510, long pass (LP) 520; Zeiss] were used in conjunction with a bolus injection of fluorescein isothiocyanate (FITC)-dextran (molecular mass 150 kD, 15 mg/kg body weight intravenously; Sigma) before injection of fluorescently labelled liposomes. Microscopic images were acquired and recorded on videotape. Data analysis was performed off-line using a computer-assisted analysis system (CAP-Image, Dr. Zeintl, Heidelberg, Germany).

Animals were randomly assigned to groups of 8 healthy or AIA animals. Fluorescently Rhodamine labelled cationic, neutral or anionic labelled liposomes, prepared as described by Krasnici et al. {Krasnici, 2003 #22}, were injected i.v. at a volume of 2 ml/kg. The relative fluorescence at three defined synovial vessel areas, showing a normal blood flow, was determined for several time points after injection, by comparison of the absolute fluorescence in the selected vessel area to an control background area outside of the vessel.

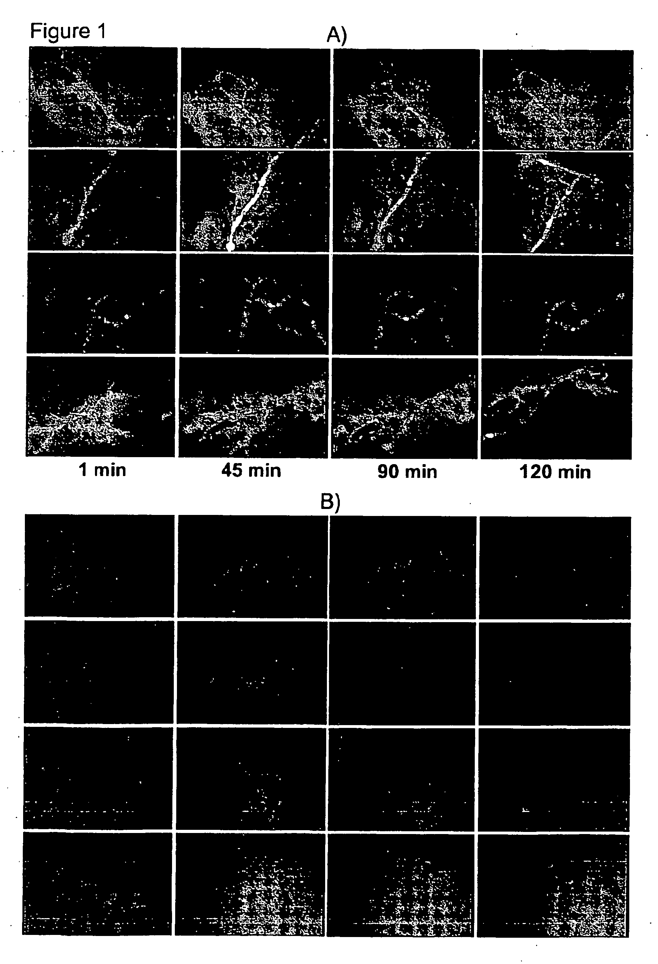

Figure 1 shows that cationic fluorescently labelled liposomes enriched at the synovial vessels of AIA mice. A clear fluorescent signal at the synovials vessel was already visible 1 min after injection of cationic fluorescently labelled liposomes, increasing at 45 min and was detectable up to 120 min (Fig. 1A). In contrast to this, hardly any fluorescent signal was detected at the synovial vessels of untreated healthy mice (Fig. 1B).

Figure 2 shows the quantification of the fluorescence kinetics of cationic, neutral and anionic fluorescently labelled liposomes binding to synovial vessels of AIA or untreated mice. Cationic Liposomes enriched more than threefold in the synovial vasculature of AIA mice compared to that of healthy mice. The maximum binding (measured as relative fluorescence, Fₘₐₓ=142 RFU) was already achieved 5 min after liposome application (tₘₐₓ) and dropped to about 50% after 100 min (t_{1/2}). In healthy mice, all of these values were lower (Fₘₐₓ=48 RFU, tₘₐₓ=15 min and t_{1/2} = 60 min). In contrast to this neutral and anionic liposomes show a very similar binding profile when comparing healthy and AIA animals. The Fₘₐₓ values are close to or lower compared to the cationic liposomes (between 109 RFU and 133 RFU) with a tₘₐₓ at 5 or 10min. Lower values for t_{1/2} (between 20 min and 45 min) for the non cationic liposomes compared to cationic liposomes in AIA animals (60min) demonstrate, that the binding of the non cationic liposomes is more transient and at 120 min after injection binding of non cationic liposomes is hardly observed in healthy or AIA animals. Cationic liposomes showed a lower binding capacity to synovial vessels of normal animals when compared to neutral or anionic liposomes.

Figure 3 demonstrates the selectivity for binding of cationic liposomes to the synovial vessels of AIA animals, resulting in a maximal 3.7 fold enrichment when compared to healthy animals. The selectivity ratio was defined by the ratio of the fluorescence signal in AIA mice compared to the fluorescence signal in healthy animals. This value varied between 2.6 and 3.7 in the first hour after application and stayed constant around 3.7 until 2h at the end of the study. This is clearly different to the non-cationic liposomes where only maximal ratios < 1.8 were observed and at the most time points the ratio was at or below 1, demonstrating that these liposomes did not enrich at synovial vessels of AIA animals.

### Example 2: In vivo binding kinetics of TNF-alpha induced binding of Rhodamine labeled cationic liposomes to subcutaneous tissues of Syrian golden hamsters

Experiments were carried out as described by Krasnici et al. {Krasnici, 2003 #22} using male Syrian golden hamsters (60-70 g b.w.) purchased from Charles River according to institutional and governmental guidelines. The animals were housed in single cages and had free access to tap water and standard laboratory food throughout the experiments.

To permit quantitative fluorescence analysis of tissue, a dorsal skinfold chamber preparation consisting of 2 symmetrical titanium frames was surgically implanted as described earlier in detail {Asaishi, 1981 #23} {Endrich, 1980 #24}.

All surgical procedures were performed under anesthesia with ketamine (100 mg/kg b.w. i.p, Ketavet; Parke-Davis, Berlin, Germany) and xylazine (10 mg/kg b.w. i.p, Rompun; Bayer, Leverkusen, Germany).

Permanently indwelling fine polyethylene catheters (PE10, inner diameter 0.28 mm) were implanted into the right jugular vein. Following a recovery period of at least 24 hr from anesthesia, chamber and catheter implantation, preparations fulfilling the criteria of microscopically intact microcirculation have been used for further investigations. For detection of rhodamine-liposomes-induced fluorescence in striated skin muscle layer, the awake chamber-bearing hamster was immobilized in a Perspex tube on a specially designed stage (Effenberger, Munich, Germany) under a Leitz macroscope (Type 307-143003/514660, Leitz, Munich, Germany). Autofluorescence of chamber tissue was determined by intravital microscopy prior to intravenous injection of liposomes (5 mg total lipid/kg b.w.) and subtracted from the respective fluorescence images. At 0.5, 1, 2, 3, 5, 10, 15, 20, 30, 45, 60, 120,180, 240 and 360 min after injection, fluorescence was registered in tumor and surrounding host tissue. Fluorophores were excited at 530-560 nm for 2 sec with a power density of 200-300 µW cm² (100 W, HBO mercury lamp). Emission fluorescence was detected above 580 nm and acquired by a silicon-intensified target video. camera (C2400-08, Hamamatsu, Herrsching, Germany). The camera setup was previously calibrated for linearity. Fluorescence images were digitized on-line by an image analysis system (KS400, Zeiss, Jena, Germany) and stored on hard disk. Spatial inhomogeneities of camera or illumination were compensated by shading correction. Densitometrical measurement of fluorescence intensities was performed off-line after correction for background fluorescence by subtraction of tissue autofluorescence. Fluorescence values are given in percentages of the solid reference fluorescence signal (% standard) inserted into each chamber (ImpregumF; ESPE, Seefeld, Germany). The geometric resolution of all images was 512 x 512 pixels at a total densitometric resolution of 255 gray values. Fluorescence was determined in regions of interest (ROI) by densitometric measurement. ROI were selected and set in the epi-illumination image of each chamber preparation prior to injection of fluorescently labeled liposomes.

Hamsters were assigned to the experimental groups of 6 animals. 40 µl of NaCl solution or 40 µl TNF-alpha solution (50IU/µl purchased from Roche) per animal were applied on the striated skin muscle layer of the chamber preparation. Cationic Rhodamine labelled liposomes were produced as described by Krasnici et al. {Krasnici, 2003 #22}.

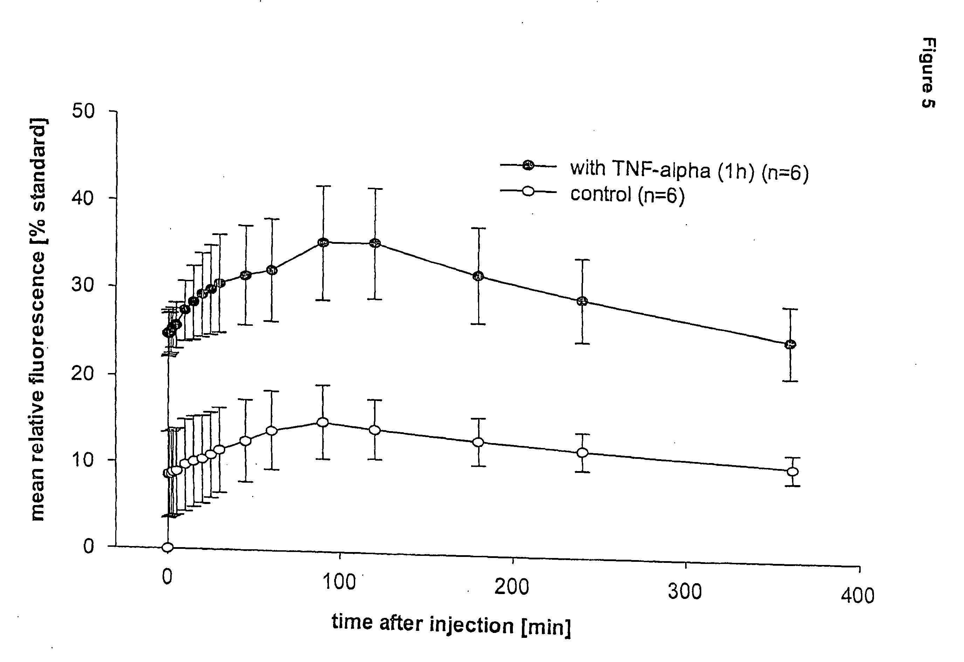

Figure 5 shows that an inflammatory stimulation with TNF-alpha is sufficient for targeting of cationic fluorescently labelled liposomes to inflamed vessels of the striated skin muscle layer. One hour after local superfusion of subcutaneous vessels of the striated skin muscle layer with 2000 IU of TNF-alpha in a dorsal skinfold chamber, fluorescence signal from cationic liposomes could be detected in microvessels of striated skin muscle. The fluorescence signal showed maximum values around 1h after liposome injection and was detected until the end of the experiment 6h after liposome injection. In contrast to this vessel areas superinfused with control NaCl solution, showed only a 60 %-70 % lower fluorescence signal over the whole observation time.

### Example 3: In vitro determination of the inhibitory activity of EndoTAG-1 and EndoTAG-placebo on IL-6- and IL-8-release from HUVEC stimulated with TNF-alpha

The pro- and/or anti-inflammatory activity of liposomes formulations such as EndoTAG-1 and EndoTAG-placebo on endothelial cells can be assessed by analysing inflammatory cytokines that are released by HUVEC in the growth culture medium after stimulation with these drugs. The higher the anti-inflammatory activity of these liposomes, the lower is the amount of IL-6 and IL-8 release from stimulated cells.

### Experiment:

Primary HUVEC (passages 2 to 4; 1 x 10e4/well) are grown overnight into 500 µl EGM2 full medium (Endothelial growth cell medium containing 5 % FBS) in 24 well plates. Culture medium is removed and 500 µl of fresh cultured medium containing 1, 50, 100 or 500 nM EndoTAG-1 (DOTAP 50%/DOPC47%/paclitaxel 3%) or EndoTAG-placebo (DOTAP 50%/DOPC 50%) either in EGM2 full medium and EGM2 Low medium (Endothelial growth cell medium containing 0.5% FBS) is added. EndoTAG-1 was prepared by the injection method as described in WO 2004/002468 by Mundus et al.. Supernatant was harvested after 48 hrs and amount of IL-8 and IL-6 cytokines were measured using "BD Cytometric Bead Array". Measurement is done in triplicates.

### Result:

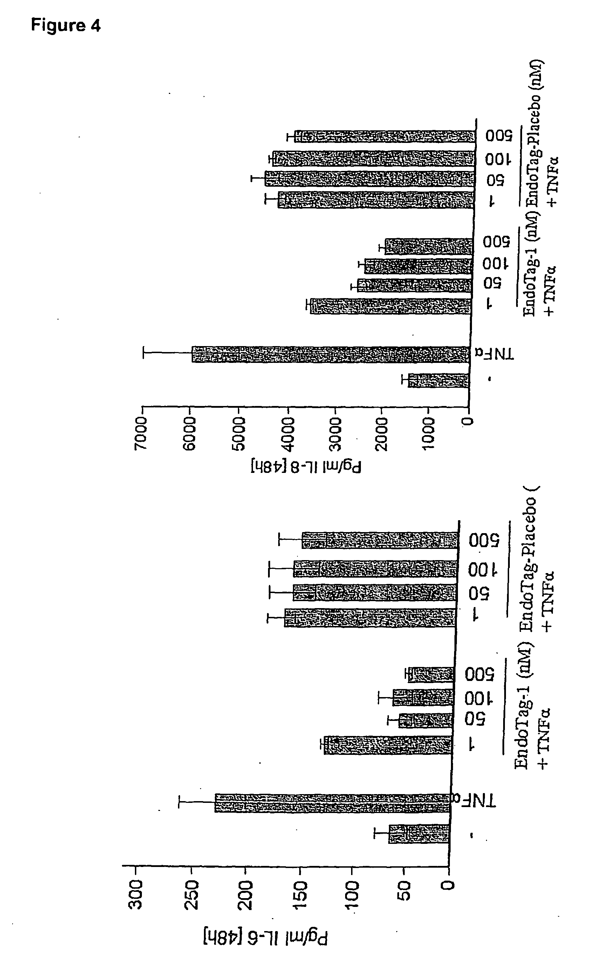

Stimulation of HUVEC with physiologically relevant concentration of inflammatory cytokines such as TNF-alpha (30 U/ml) in presence of increasing concentration of EndoTAG-1 or EndoTAG-placebo clearly show an inhibition of IL-6 and IL-8 released by HUVEC. Notably, at the lowest concentration of EndoTAG-1 used in this assay (1nM EndoTAG-1) 37 % inhibition of IL-8 (Fig. 4, right part) and 35 % IL-6 (Fig. 4, left part) release can be observed. A concentration of 50 nM of EndoTAG-1 was sufficient to get up to 71 % inhibitory activity whereas a ten fold higher concentration of EndoTAG-1 (up to 500 nM) does not show a significant increase of the inhibitory activity as compare to 50 nM concentration (Fig. 4). Under the same experimental conditions EndoTAG-placebo shows up to 34 % inhibition of IL-8 (Fig. 4, right part) or 21 % IL-6 (Fig. 4, left part) release by HUVEC.

### Example 4: Therapeutic effect of EndoTAG-1 on rat Carrageenan-induced paw inflammation

Male Sprague Dawley rats with an avarage weight of 248 grams at arrival were purchased from Harlan Inc. and housed in isolated cages under save environmental conditions (3-4 rats per cage, 22° C, 30-70 % humidity and 12 h light/dark cycle) with food and water ad libitum. Animals were acclimated for 3 days prior to being placed on study. Experimental design was reviewed and approved by local government.

Animals (6/group), dosed with test articles by intravenous injection (5 ml/kg) into the tail vein, were injected with 100 µl of 1.2 % carrageenan into the right hind footpad and were then euthanized at four hours post injection for evaluation of paw swelling, based on paw weight determination. EndoTAG-1 (DOTAP 50%/DOPC47%/paclitaxel 3%) or EndoTAG-placebo (DOTAP 50%/DOPC 50%) have a lipid content of 10 mM in a 10% m/m trehalose dihydrate solution and were prepared as described in WO 2004/002468 by Mundus et al.. Taxol® was a CremophorEL formulated Paclitaxel purchased from Bristol-Myers Squibb. Drug solutions were administered i.v. with slow bolus in a volume of 10 µl/g. Animals were dosed 30 minutes post Carrageenan injection as indicated below:

| **Group name** | **Animal no.** | **Treatment** | **Appl. vol. [ml/kg]** | **Dose mg/kg** |
|---|---|---|---|---|
| Disease control | 3 | 9.8 % Trehalose | 5 | -- |
| EndoTAG-1 placebo | 6 | EndoTAG-1 placebo | 5 | -- |
| Taxol® | 6 | Taxol | 5 | 1,28* |
| EndoTAG-1 | 6 | EndoTAG-1 | 5 | 1,28* |

| | | | | |
|---|---|---|---|---|
| * given as paclitaxel concentration | | | | |

Figure 6 shows that treatment with EndoTAG-1 or EndoTAG-1 placebo had a clear therapeutic effect on rat Carrageenan-induced paw inflammation, measured as decrease in paw weight. Treatment with EndoTAG-1 30 minutes post Carrageenan injection significantly reduced left/right (untreated/inflamed) paw weight differences compared to the post-injection trehalose group by 38 %, as well as reducing the weight difference compared to the Taxol® group by 33 %. EndoTAG-1 placebo treatment significantly reduced weight difference by 28 %, while Taxol® given as 1.28 mg/kg/day Paclitaxel treatment did not. The effects of Taxol® given as 1.28 mg/kg/day paclitaxel alone and EndoTAG-1 placebo alone were generally aditive for the EndoTAG-1 given as 1.28 mg/kg/day paclitaxel.

### Example 5: Therapeutic effect of EndoTAG-1 on collagen induced arthritis in DBA/1 mice

Male DBA/1 mice with an age of ten weeks at arrival were purchased from Taconic Europe and housed in isolated cages under save environmental conditions (5 mice per cage, 22°C, 30-70 % humidity and 12 h light/dark cycle) with food and water ad libitum. Experimental design was reviewed and approved by local government.

Mice were assigned to the experimental groups acclimated at least 7 days before induction of collagen induced arthritis. Animals were anesthetized intraperitoneally (i.p.) with a mixture of Xylazin and Ketamin. Each animal obtained 4 ml/kg as application volume. 0.5 ml Rompun (20 mg/ml Xylazin) and 1 ml Ketamin (100 mg/ml Ketamin) were mixed (1:2) and diluted 1:4 in 4.5 ml 0.9 % NaCl. 2. For the Collagen Type II-CFA emulsions, Collagen (bovine, 4 mg/ml) was mixed 1:1 with complete Freund's adjuvant (CFA) by repeated passage through a flexible tube connecting two 1-ml syringes (syringe-syringe-method). This mixture was kept on ice and were applied within 4 hours after preparation. For the immunization 100 µl Collagen/CFA-emulsion was injected subcutaneously at the base of the tail. The injection of Collagen/CFA-emulsion was done once on day 0 and once as a booster immunization on day 21. After two weeks the animals were controlled for symptoms of collagen induced arthritis (CIA) by joint scoring three times a week. To document the progression of arthritis for each animal, grades of arthritis was classified as arthritis index (AI) by score 0-4 per joint. Symptoms were defined by the following symptom-list:
0: normal
1: mild swelling and erythema/reddening
2: moderate swelling and erythema
3: severe swelling and erythema of entire paw including digits
4: ankylosis

Treatment with drugs started day 7. Drugs were given three times a week (Mo, Wed, Fri) for the following four weeks. EndoTAG-1 (DOTAP 50%/DOPC47%/paclitaxel 3%) or EndoTAG-placebo (DOTAP 50%/DOPC 50%) have a lipid content of 10 mM in a 10 % m/m trehalose dihydrate solution and were prepared as described in WO 2004/002468 by Mundus et al. Taxol® was a CremophorEL formulated Paclitaxel purchased from Bristol-Myers Squibb. Drug solutions were administered i.v. with slow bolus in a volume of 10 µl/g. Animals of each group were treated according to the table.

| **Group name** | **Animal no.** | **Treatment** | **Appl. vol. [ml/kg]** | **Number of appl. per week** | **Dose mg/kg** |
|---|---|---|---|---|---|
| Disease control | 15 | 9.8 % Trehalose | 10 | 3 | -- |
| EndoTAG-1 placebo | 15 | EndoTAG-1 placebo | 10 | 3 | -- |
| Taxol® | 15 | Taxol® | 10 | 3 | 2,56* |
| EndoTAG-1 | 15 | EndoTAG-1 | 10 | 3 | 2,56* |

| | | | | | |
|---|---|---|---|---|---|
| • given as paclitaxel concentration • | | | | | |

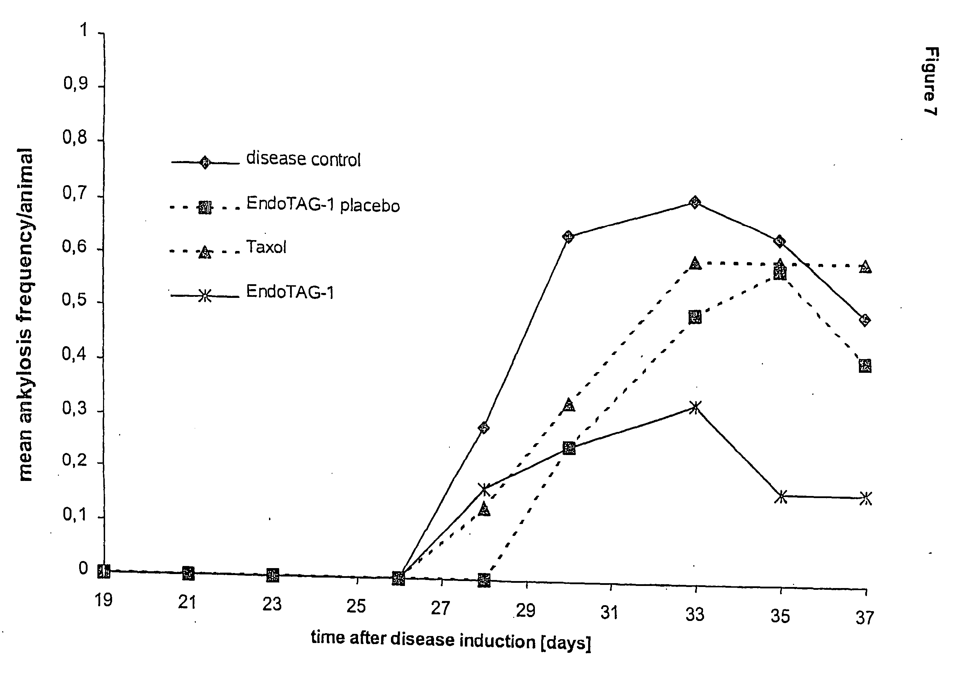

As shown in Figure 7 EndoTAG-1 was clearly efficacious in the prophylactic treatment of arthritic paws when compared to the trehalose disease group, measured as a decrease in the frequency of paws showing ankylosis, being the most severe grade of arthritis. This effect was observed from day 28 on and was visible until the end of the study. At day 33 disease was most advanced with the highest ankylosis frequency observed in the disease group. At that time point the antiarthritic efficacy of the drugs was in the order EndoTAG-1 > EndoTAG-1 placebo > Taxol®. Paclitaxel given as liposomal EndoTAG-1 strongly reduced the ankylosis frequency and was far more effective than free paclitaxel given as Taxol®.

### Example 6: Therapeutic effect of EndoTAG-1 on collagen induced arthritis (CIA) in B10RIII mice

Male B10RIII mice with an age between 7-9 weeks at arrival were purchased from Jackson Laboratories and housed in isolated cages under save environmental conditions (4-5 mice per cage, 22°C, 30-70 % humidity and 12 h light/dark cycle) with food and water ad libitum. Experimental design was reviewed and approved by local government.

Animals were anesthetized with Isoflurane and injected with 150 µl of Freund's Complete Adjuvant (Difco) containing bovine type II collagen (Elastin Products, Owensville, MO) (2 mg/ml) and supplemental mycobacterium tuberculosis H37 RA (Difco) at the base of the tail on days 0 and 15. Male B10RIII mice with developing type II collagen arthritis were randomized into groups that received once daily i.v. treatments.

After two weeks the animals were controlled for symptoms of collagen induced arthritis (CIA) by joint scoring every day. To document the progression of arthritis for each animal, grades of arthritis were classified as arthritis index (Al) by score 0-5 per joint. Symptoms were defined by the following symptom-list:
0=normal
1=1 hind or fore paw joint affected or minimal diffuse erythema and swelling
2=2 hind or fore paw joints affected or mild diffuse erythema and swelling
3=3 hind or fore paw joints affected or moderate diffuse erythema and swelling
4= Marked diffuse erythema and swelling, or =4 digit joints affected)
5=Severe diffuse erythema and severe swelling entire paw, unable to flex digits)

Treatment with drugs started when animals showed first clinical signs of arthritis (at least one paw with arthritis score>0). From this day of enrollment (day 0) drugs were given every second day until day 11 and clinical symptoms were monitored daily. EndoTAG-1 (DOTAP 50%/DOPC47%/paclitaxel 3%) or EndoTAG-placebo (DOTAP 50%/DOPC 50%) have a lipid content of 10 mM in a 10% m/m trehalose dihydrate solution and were prepared as described in WO 2004/002468 by Mundus et al.. Enbrel (Etanercept) is a dimeric fusion protein consisting of the extracellular ligand-binding portion of the human 75 kilodalton (p75) tumor necrosis factor receptor (TNFR) linked to the Fc portion of human IgGl and was purchased from AMGEN. Drug solutions were administered i.v. with slow bolus in a volume of 10 µl/g. Animals of each group were treated according to the table.

| **Group name** | **Animal no.** | **Treatment** | **Appl. vol. [ml/kg]** | **Dose mg/kg** |
|---|---|---|---|---|
| Disease control | 15 | 9.8 % Trehalose | 10 | -- |
| EndoTAG-1 placebo | 15 | EndoTAG-1 placebo | 10 | -- |
| Taxol® | 15 | Taxol® | 10 | 2,56* |
| EndoTAG-1 | 15 | EndoTAG-1 | 10 | 2,56* |
| Enbrel | 10 | Etanercept | 10 | 10 |

| | | | | |
|---|---|---|---|---|
| * given as paclitaxel concentration | | | | |

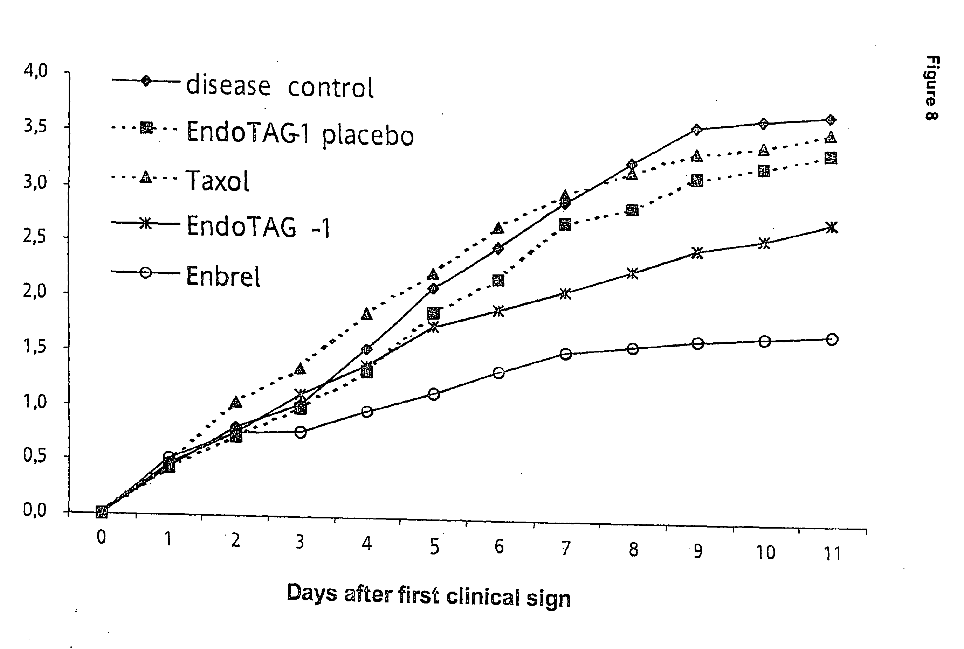

As shown in Figure 8 EndoTAG-1 showed a clear efficacy in the therapeutic treatment of arthritic paws when compared to the trehalose disease group, measured as a decrease in the mean clinical arthritis score. This effect is observed from day 6 after enrollment on and was visible until the end of the study at day 11. At the endpoint the antiarthritic efficacy of the drugs is in the order Enbrel > EndoTAG-1 > EndoTAG-1 placebo and Taxol®. Paclitaxel given as liposomal EndoTAG-1 strongly reduced the mean clinical arthritis score and was far more effective then the free paclitaxel given as Taxol®. Also liposomes comprising no further active agent (EndoTAG-1 placebo) improved the clinical score in comparison to Taxol®.

### Example 7: Therapeutic effect of EndoTAG-1 on antigen induced arthritis in C57Black6 mice

Female C57Black6 mice with an age of 8-10 weeks at arrival, weighing 15-17 grams, were purchased from Chrales River, Germany and housed in isolated cages under save environmental conditions (5 mice per cage, 22° C, 30-70 % humidity and 12 h light/dark cycle) with food and water ad libitum. Experimental design was reviewed and approved by local government.

Mice were assigned to the experimental groups acclimated at least 14 days before induction of antigen-induced arthritis (AIA). Animals were anesthetized with Isoflurane and immunized by a subcutaneous injection of 100 µg of methylated bovine serum (mBSA; Sigma, Deisenhofen Germany), dissolved in 50 µl of Freund's Complete Adjuvant (Sigma) and supplemented with mycobacterium tuberculosis H37 RA (Difco) and an additional intraperitoneal injection of 2*10⁹ heat-killed Bordetella pertussis(DSMZ, Germany) on days-21 and -14 prior to induction of arthritis as described by Brackertz et al. {Brackertz, 1977 #19} . Arthritis was induced on day 0 by injection of 100 µg mBSA in 50 µl saline into the left knee joint using a sterile 33-gauge microcannula.

Treatment with drugs started day 0. Drug solutions were administered i.v. with slow bolus in a volume of 10 µl/g. Animals of each group were treated according to the table at day 0, 2, 4, 7, 9 and 11 and knee joint diameters were determined for arthritis quantification.

| **Group name** | **Animal no.** | **Treatment** | **Appl. vol. [ml/kg]** | **Number of appl. per week** | **Dose mg/kg** |
|---|---|---|---|---|---|
| Disease control | 15 | 9.8 % Trehalose | 10 | 3 | -- |
| EndoTAG-1 placebo | 15 | EndoTAG-1 placebo | 10 | 3 | -- |
| Taxol® | 15 | Taxol® | 10 | 3 | 2,56* |
| EndoTAG-1 | 15 | EndoTAG-1 | 10 | 3 | 2,56* |

| | | | | | |
|---|---|---|---|---|---|
| * given as paclitaxel concentration | | | | | |

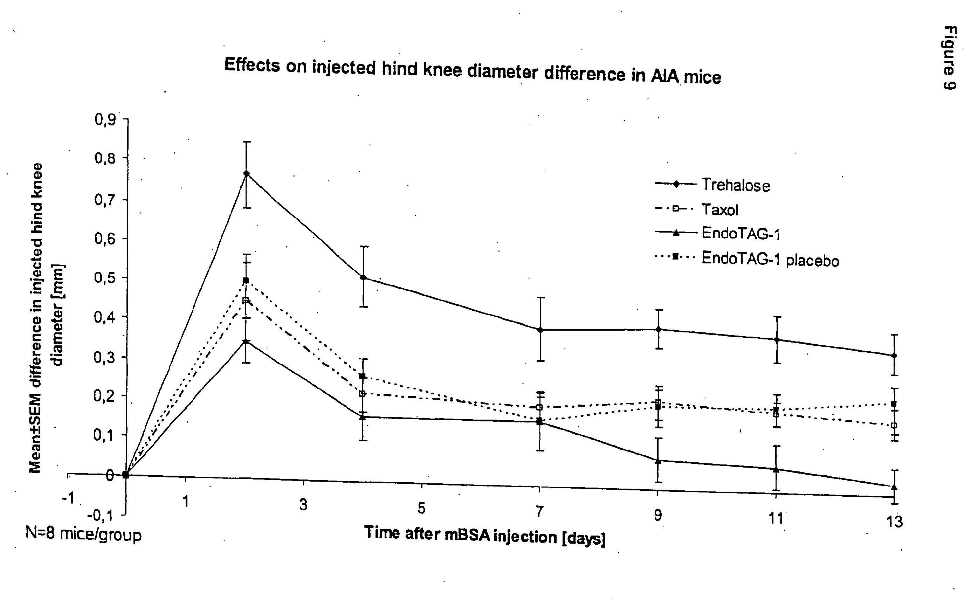

As shown in Figure 9 EndoTAG-1 showed a clear efficacy in the therapeutic treatment of arthritic knees from AIA animals when swelling of the diseased knee was compared to the trehalose treated disease control group. This effect was observed from day 2 after disease induction and was visible until the end of the study at day 13. At the endpoint the antiarthritic efficacy of the drugs is in the order EndoTAG-1 > EndoTAG-1 placebo and Taxol®. Paclitaxel given as liposomal EndoTAG-1 reduced the knee swelling by 93% compared to the disease control and was far more effective then the free paclitaxel given as Taxol®. Treatment with EndoTAG-1 finally resulted in knee diameters that were nearly identical to the diameter before disease induction.

### Example 8: Therapeutic effect of EndoMTX on collagen induced arthritis in DBA/1 mice

Male DBA/1 mice with an age of ten weeks at arrival were purchased from Taconic Europe and housed in isolated cages under save environmental conditions (Five mice per cage, 22°C, 30-70% humidity and 12h light/dark cycle) with food and water ad libitum. Experimental design was reviewed and approved by local government.

Mice were assigned to the experimental groups acclimated at least 7 days before induction of collagen induced arthritis. Animals were anesthetized intraperitoneally (i.p.) with a mixture of Xylazin and Ketamin. Each animal obtained 4 ml/kg as application volume. 0.5 ml Rompun (20 mg/ml Xylazin) and 1 ml Ketamin (100 mg/ml Ketamin) were mixed (1:2) and diluted 1:4 in 4.5 ml 0.9 % NaCl. 2. For the Collagen Type II-CFA emulsions, Collagen (bovine, 4 mg/ml) was mixed 1:1 with CFA by repeated passage through a flexible tube connecting two 1-ml syringes (syringe-syringe-method). This mixture was kept on ice and will be applied within 4 hours after preparation. For the immunization 100 µl Collagen/CFA-emulsion was injected subcutaneously at the base of the tail. The injection of Collagen/CFA-emulsion was done once on day 0 and once as a booster immunization on day 21. After two weeks the animals were controlled for symptoms of collagen induced arthritis (CIA) by joint scoring three times a week. To document the progression of arthritis for each animal, grades of arthritis are classified as arthritis index (AI) by score 0-4 per joint. Symptoms were defined by the following symptom-list.
0: normal
1: mild swelling and erythema/reddening
2: moderate swelling and erythema
3: severe swelling and erythema of entire paw including digits
4: ankylosis
Treatment with drugs started day 15. Drugs were given three times a week (Mo, Wed, Fri) for the following three weeks. EndoMTX (10 mM DOTAP; 1,1 mM methotrexate) can be prepared by co-extrusion as described in Example 10. Drug solutions were administered i.v. with slow bolus in a volume of 10 µl/g. Animals of each group were treated according to the table.

| **Group name** | **Animal no.** | **Treatment** | **Appl. vol. [ml/kg]** | **Number of appl. per week** | **Dose mg/kg** |
|---|---|---|---|---|---|
| Disease control | 15 | 9.8 % Trehalose | 10 | 3 | -- |
| EndoMTX placebo | 15 | EndoMTX placebo | 10 | 3 | -- |
| MTX | 15 | Methotrexate | 10 | 3 | 5,0 |
| EndoMTX | 15 | EndoMTX | 10 | 3 | 5,0* |

| | | | | | |
|---|---|---|---|---|---|
| * given as methotrexate concentration | | | | | |

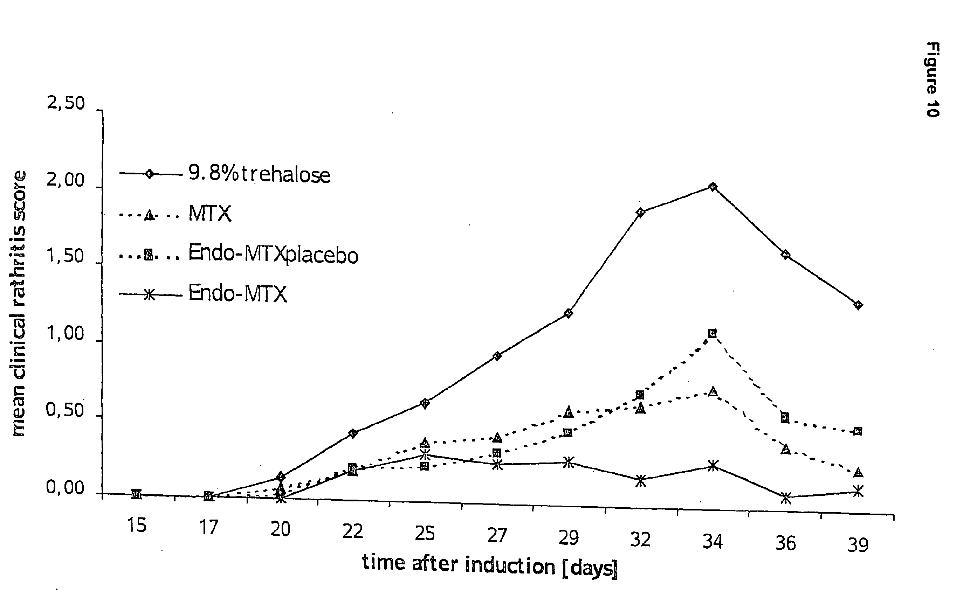

As shown in Figure 10 EndoMTX showed a clear efficacy in the treatment of the arthritic paws when compared to the trehalose disease group, measured as a decrease in the mean clinical arthritis score. This effect is observed from day 28 on and was visible until the end of the study. At day 34 disease was most advanced with the highest mean score value observed for the disease group. At this time point the antiarthritic efficacy of the drugs is in the order EndoMTX> EndoMTX placebo > MTX. Methotrexate given as liposomal EndoMTX strongly reduced the mean clinical arthritis score and was more effective then the free Methotrexate or the EndoMTX placebo.

### Example 9: Histologic assessment and immunostaining of non proliferating and proliferating vessel endothelial cells in joint tissue.

Analysing the percentage of proliferating versus non-proliferating endothelial cells in joint tissue sections of rheumatoid arthritis mouse models can assess the proliferation activation phenotype of vessels in these tissue sections in response to candidates' drug application. A reduced number of proliferating endothelial cells and/or vessel density suggest an anti-angiogenic and/or anti-inflammatory activity of the drug.

### Experiment:

Front paws, hind paws and hind knees of mice (Balb/C; DBA-1; B10RIII) are fixed for 24 hours in 4 % buffered formalin and decalcified in 0.5 M EDTA solution (pH = 7.1) at room temperature until bones are pliable. Joint tissues contain calcium deposits which are extremely firm and which will not section properly with paraffin embedding owing to the difference in densities between calcium and paraffin. This calcium must be removed prior to embedding to allow sectioning. Serial paraffin sections of all paws and knees are done at 2 µm, stained with hematoxylin and eosin (H&E) and safranin O and analysed by phase-contrast microscopy.

For Immunostaining of endothelial cells, dewaxed and ethanol-dehydrated tissue sections are blocked with 3 % H₂O₂, rinsed and boiled three times in a 800W microwave in 10mM sodium citrate buffer (pH = 6) for antigen retrieval.

Tissue sections are blocked for 30 min in PBS containing 5 % donkey (for staining with goat anti-mouse CD31 primary antibody) or goat (rabbit anti-mouse PCNA primary antibody) serum, followed by incubation overnight at 4° C with the following antibodies: goat anti-mouse CD31 (dilution: 1:1500, clone M-20, Santa Cruz, Santa Cruz, CA, USA) or rabbit anti-mouse PCNA (dilution: 1:100, polyclonal, Abcam, Cambridge, UK). After rinsing, sections are incubated with biotinylated species-specific anti-IgG secondary antibodies (donkey anti-goat IgG, Santa Cruz; goat anti-rabbit IgG, Vector) for 1 h at 20° C. Antigen-positive cells are stained brown after incubation with the appropriate Vectastain ABC reagent (Vector, Burlingame, CA, USA) and 3,3'-diaminobenzidine (Sigma-Aldrich, Munich, Germany).

Synovial inflammation, bone erosion, cartilage destruction and quantification of vessel density and proliferating cells is done using a Zeiss microscope (Zeiss, Goettingen, Germany) equipped with a digital camera and image analysis system (Spot model 2.3.1, Diagnostic Instruments, Sterling Heights, MI, USA; MetaVue, Universal Imaging Corporation, Chicago, IL, USA).

### Result:

Serial tissue sections are stained with CD31 (endothelial cell marker) and PCNA or Ki67 (proliferation markers) as describe above. i) The lower the ratio of Ki67+ and/or PCNA+ and CD31+ cells (proliferating endothelial cells) over PCNA- and/or Ki67- and CD31+ cells (non proliferating endothelial cells) the higher is the anti-angiogenic/anti-inflammatory activity of the drug tested. ii) The lower is the number of vessels in a tissue section the higher is the anti-angiogenic/anti-inflammatory activity of the drug tested. iii) Validation of an anti- or pro-inflammatory activity of the drug required statistically significant differences of these ratios as compared to a placebo.

The rate of apoptotic cells will be determined by staining the well-characterized pro-apoptotic marker protein p53. The lower the ratio of CD31+/P53- (non-apoptotic endothelial cells) over CD31+/P53+ (apoptotic endothelial cells) in a given tissue section the higher is the apoptotic activity of the drug tested.

### Example 10: Preparation of cationic liposomal methotrexate preparations

### Preparation of Endo-MTX Formulations by Co-extrusion (CF004)

20 mM DOTAP liposomes (20 ml) were prepared by the lipid film method as described in WO 2004/002468 by Mundus et al., rehydration was performed with 10 % trehalose. Next, the liposomes were mixed with 20 ml of a sodium MTX solution (2.2 mM, prepared from diluting a 220 mM sodium MTX solution with 10% trehalose). The resulting solution (theoretical concentration now 10 mM DOTAP and 1.1 mM MTX) was extruded 5 times through a polycarbonate membrane with 200 nm pore size.
Subsequently, HPLC and PCS analytics were performed. DOTAP: 8.4 mM, MTX 1.14 mM (for HPLC methods, see below). Zₐᵥₑ = 156 nm, PI 0.29 Other analytics: zeta potential: 59.3 mV ± 1.4 mV (after 1:10 dilution in a solution containing 50 mM KCI and 10 % trehalose)
MTX release from liposome was determined by centrifugation through Centricon tube, (MWCO = 30,000, 4500 rcf, 180 min) and was found to be 1.4 % of the MTX concentration.
The formulation is stable at 4° C for at least 16 weeks.

### Preparation of PEGylated Endo-MTX Formulations by Co-extrusion (CF005)

20 mM DOTAP/PEG-DOPE liposomes with a molar ratio 95/5 mol% (total volume of 20 ml) were prepared by the lipid film method as described in WO 2004/002468 by Mundus et al., rehydration was performed with 10 % trehalose. Next, the liposomes were mixed with 20 ml of a sodium MTX solution (2.2 mM, prepared from diluting a 220 mM sodium MTX solution with 10 % trehalose). The resulting solution (theoretical concentration now 9.5 mM DOTAP, 0.5 mM PEG7DOPE, 1.1 mM MTX) was extruded 5 times through a polycarbonate membrane with a pore size of 200 nm. Subsequently, HPLC and PCS analytics were performed. DOTAP 8.83 mM, MTX 1.02 mM (for HPLC methods, see below). Zₐᵥₑ = 161 nm, PI 0.225 Other analytics: zeta potential: -2.4 mV ± 0.5 mV (after 1:10 dilution in a solution containing 50 mM KCI and 10 % trehalose)
MTX release from liposome was determined by centrifugation through Centricon tube, (MWCO = 30,000, 4500 rcf, 180 min): 2.7 %
The formulation is stable at 4 C for at least 16 weeks.

### Preparation of Endo-MTX Formulations by Mixing (MRa0036)

20 mM DOTAP liposomes (20 ml) were prepared by the lipid film method as described in WO 2004/002468 by Mundus et al., rehydration was performed with 10 % trehalose. Then, the liposomes were extruded 5 times through 200 nm membrane (polycarbonate). Next, the resulting SUV suspension was mixed with 20 ml of a sodium MTX solution (2.2 mM, prepared from diluting a 220 mM sodium MTX solution with 10 % trehalose). The resulting solution was 10 mM DOTAP and 1.1 mM MTX).
Subsequently, HPLC and PCS analytics were performed. DOTAP: 9.6 mM, MTX 1.14 mM (for HPLC methods, see below). Zₐᵥₑ = 145 nm, PI 0.373 Other analytics: zeta potential: 50 mV ± 0.7 mV (after 1:10 dilution in a solution containing 50 mM KCI and 10 % trehalose)
MTX release from liposome was determined by centrifugation through Centricon tube, (MWCO = 30,000, 4500 rcf, 180 min) = 1.4 %
The formulation is stable at 4 C for at least 16 weeks.

### Preparation of PEGylated Endo-MTX Formulations by Mixing (MRa0037)

20 mM DOTAP/PEG-DOPE liposomes with a molar ratio 95/5 mol% (total volume of 20 ml) were prepared by the lipid film method as described in WO 2004/002468 by Mundus et al., rehydration was performed with 10 % trehalose. Next, the liposomes were extruded 5 times through 200 nm membrane (polycarbonate). Then, the resulting SUVs were mixed with 20 ml of a sodium MTX solution (2.2 mM, prepared from diluting a 220 mM sodium MTX solution with 10 % trehalose), resulting in a suspension with now 9.5 mM DOTAP, 0.5 mM PEG-DOPE, 1.1 mM MTX.
Subsequently, HPLC and PCS analytics were performed. DOTAP: 9.6 mM, MTX 1.14 mM (for HPLC methods, see below). Zₐᵥₑ = 157 nm, PI 0.259 Other analytics: zeta potential: 1.1 mV ± 1.1 mV (after 1:10 dilution in a solution containing 50 mM KCl and 10 % trehalose)
MTX release from liposome was determined by centrifugation through Centricon tube (MWCO = 30,000, 4500 rcf, 180 min) = 3.6 %
The formulation is stable at 4° C for at least 16 weeks.

### Analysis of DOTAP content by HPLC

As stationary phase, a C8 column Luna 5 µ C8 (2) 100 A, 150x2 mm (Phenomenex) is used. The mobile phase is composed of water with 0.1% TFA (solvent A) and acetonitrile with 0.1% TFA (solvent B), the following gradient program is run:

| **Time (min)** | **Solv. B (%)** |
|---|---|
| 0.00 | 50.0 |
| 4.12 | 50.0 |
| 7.06 | 75.0 |
| 14.13 | 100.0 |
| 21.20 | 100.0 |
| 23.56 | 50.0 |
| 30.00 | 50.0 |

| | |
|---|---|
| column temperature: 45 °C Injektion volume: 5 µl wavelength for detektion: 205 nm run time: 30 min | |

### Analysis of methotrexate content by HPLC

An isocratic method is employed, using a C18 stationary phase (Luna 5 µ C18 (2) 100 A, 150x2 mm (Phenomenex)
The mobile phase is composed of 10 mM NH₄OAc pH: 6.0 and acetonitrile at a ratio 93/7 (v/v).
column temperature: 40 °C
Injection volume: 10 µl
wavelength for detektion: 310 nm
run time: 15 min

### Example 11: Histological assessment of therapeutic effects of EndoMTX on collagen induced arthritis (CIA) in DBA/1 mice

Animals from experiments described in Example 8 were analysed by histopathological methods for the assessment of therapeutic effects of EndoMTX. After ether anaesthesia, fore paw joints and hind paw and knee joints were taken out and the fur, skin and most of the muscle tissue were taken off.

Tissue was incubated for 24 h in 4 % buffered formalin at room temperature (RT) and subsequently inserted into 25 mL 0.5. M EDTA buffer (pH = 7.1) for 5 days (knees) or 8 days (paws), respectively. EDTA buffer was exchanged once for the knee group or twice for the paw group and joints were cut into half in medial direction (lenghtwise). The decalcification step was followed by washing twice with PBS and paraffination overnight. After that, knees and paws were blocked out separately.

Tissue from each joint sample was sectioned to 2 µm and stained with Safranin O/Fast Green (fore and hind paws) and additionally with Haemalaun (hind knees). After deparaffination of 2 µm cut sections (3 x xylol (10 min), 2 x ethanol (2 min), 1 x 90% ethanol (2 min), 1 x 80% ethanol (2 min), 1 x 70% ethanol (2 min), water (2 min)), tissue was stained for 5 min in 0.001% Fast Green, washed for 10 s in 1% acetic acid and stained again for 5 min in 0.01% Safranin O. This was followed by dehydration in twice 96% ethanol, twice 100% ethanol and twice xylol for 2 min. In the end, slides were covered with Entellan Neu.

The evaluation and scoring of joint tissue samples was assessed using a scoring system as follows. The scores of inflammation, pannus development and cartilage erosion were defined in five stages (0 to 4) by a comparative study of healthy versus arthritic mice, equally to the clinical score of mouse paws. The stages are normal, mild, moderate, marked and severe. Classification of inflammation is based on the infiltration of mononuclear cells. The highest score is characterized by the infiltration of periarticular cells and gives a rather qualitative description of affection. The scores of pannus formation stages the size of tissue development and therefore gives a rather quantitative view of inflammation. The cartilage erosion is described by the reduction of Safranin O staining in pathologic tissue in comparison with healthy cartilage tissue. The highest score represents the total loss of staining and/or bone erosion.

The scoring system is shown in the following table.

| **Score** | **Inflammation** | **Pannus development** | **Cartilage erosion** |
|---|---|---|---|
| **0** | Normal | Normal | Normal |
| **1** | Mild mononuclaer cell infiltrates | Mild formation of inflamematory tissue | Mild loss of Safranin O staining |
| **2** | Moderate cell infiltrates | Moderate formation of inflammatory tissue | Moderate loss of Safranir O staining |
| **3** | Market cell infiltrates | Market formation of inflammatory tissue; hyperplasia of the synovium, synovitis | Market loss of Safranin C staining |
| **4** | Severe cell infiltrates; periarticular infiltration | Severe formation of inflammatory tissue; synovitis; destruction of joint architecture | Severe loss of Safranin C staining; Cartilage erosio |

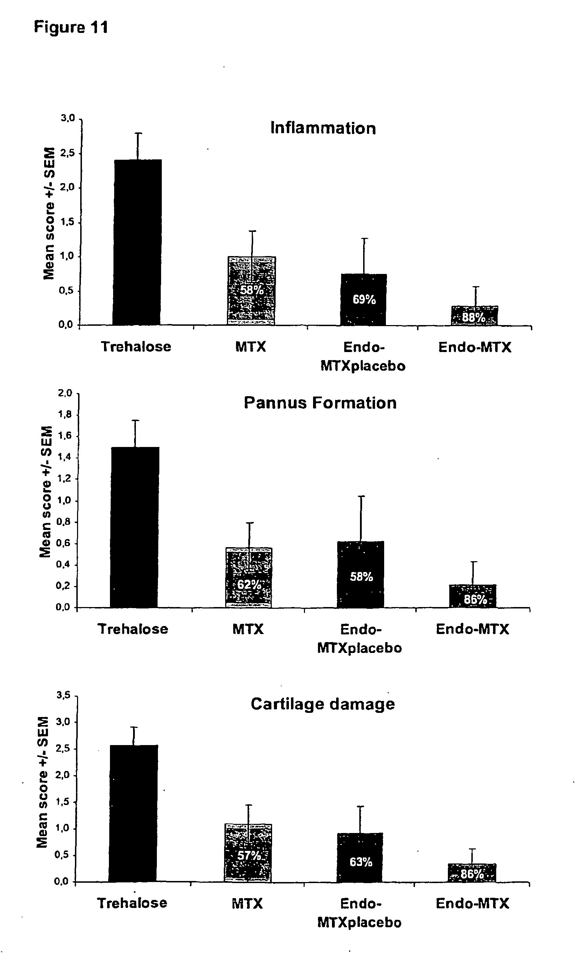

As shown in Figure 11 treatment with EndoMTX resulted in the lowest mean score of 0.29 in inflammation, 0.21 in pannus formation and 0.36 in cartilage damage, which was shown by histopathology in sections of hind knees from mice treated with EndoMTX. EndoMTX demonstrated 88 % reduction in inflammtion, 86 % reduction in pannus formation and 86 % reduction in cartilage damage compared with the Trehalose group (control).

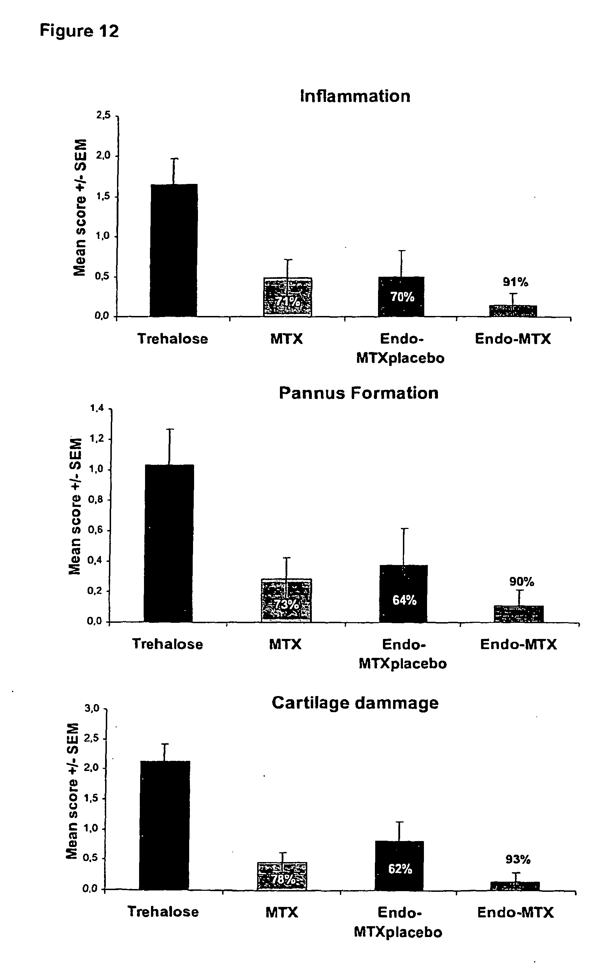

As shown in Figure 12 very similar effects were observed in all paw joints analysed.

### Example 12: Histological assessment of therapeutic effects of EndoMTX on osteoclast formation/bone destruction in collagen induced arthritis (CIA) in DBA/1 mice

Animals from experiments described in Example 8 were analysed by histopathological methods for the assessment of therapeutic effects of EndoMTX on osteoclast formation/bone destruction. As a marker for bone destruction the number of osteoclasts, the key cells responsible for bone destruction, were determined. The epiphysis of the femur and tibia of the knee joints were investigated for nucleated TRAP+ cells, counted and compared as osteoclasts per epiphysis (femur or tibia separately) and osteoclasts per knee joint (epiphyseal osteoclasts of femur and tibia together).

After ether anaesthesia, knee joints were taken out and the fur, skin and most of the muscle tissue were taken off. Tissue was incubated for 24 h in 4 % buffered formalin at RT and subsequently inserted into 25 mL 0.5 M EDTA buffer (pH = 7.1) for 5 days (knees) or 8 days (paws), respectively. EDTA buffer was exchanged once for the knee group and twice for the paw group, joints were cut into half in medial direction (lenghtwise) and decalcification step was followed by washing twice with PBS and paraffination overnight. After that, knees were blocked out.

After deparaffination of 2 µm cut sections (3 x xylol (10 min), 2 x ethanol (2 min), 1 x 90 % ethanol (2 min), 1 x 80 % ethanol (2 min), 1 x 70 % ethanol (2 min), water (2 min)), tissue was stained for 1 h at 37° C in TRAP solution (Sigma Kit 386A). This was followed by washing twice in dest. water and counterstain in Haemalaun (1:4) for 8min. After washing in running tap water for 5min and twice dest. water for 2min, sections were mounted with DAKO Faramount Aqueous.

The epiphysis of the femur and tibia of the knee joints were investigated for nucleated TRAP+ cells, counted and compared as osteoclasts per epiphysis (femur or tibia separately) and osteoclasts per knee joint (epiphyseal osteoclasts of femur and tibia together).

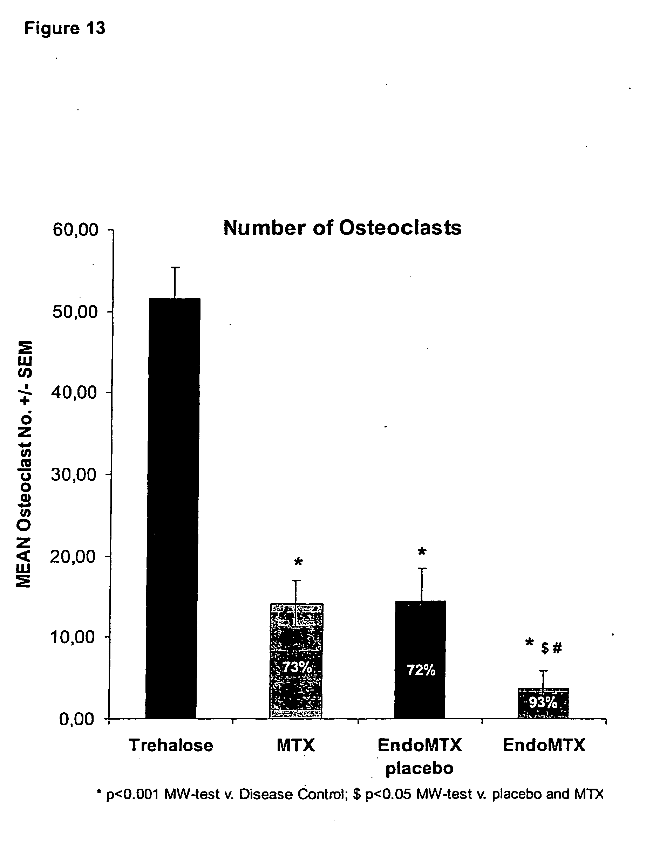

As shown in Figure 13 EndoMTX revealed the highest efficacy for the reduction of osteoclasts compared with all other groups and showed a reduction of osteoclasts of 93 % compared with the Trehalose group. Using the ranked Mann-Whittney Sum test EndoMTX was statistically significant compared with Trehalose (p<0.001), MTX and EndoMTX placebo (p<0.05), respectively.

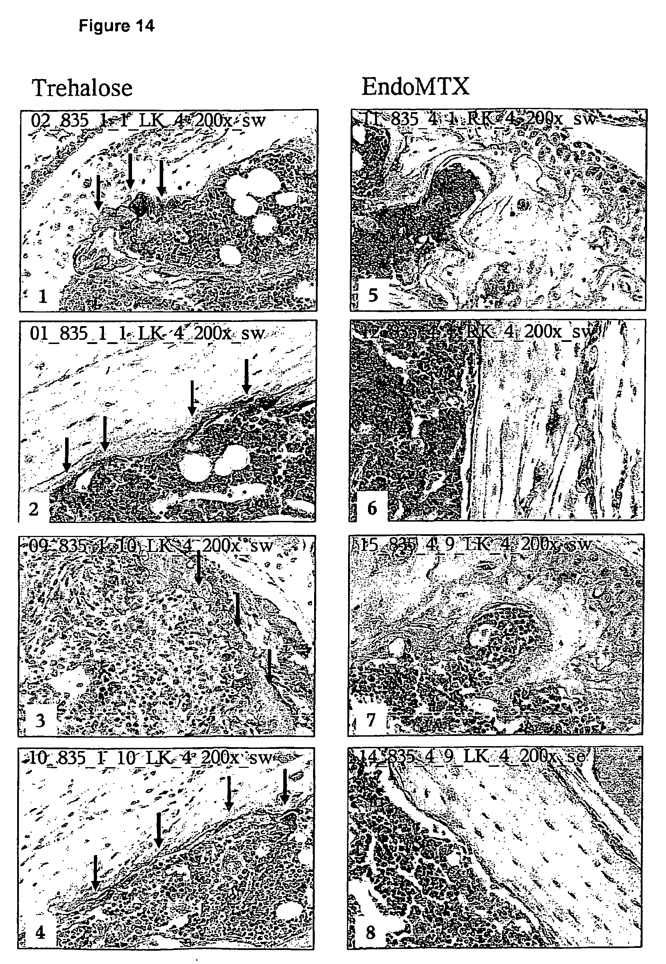

As shown in Figure 14 EndoMTX clearly reduced the bone destruction compared to the Trehalose treated animals. Sections from hind knees were stained with the tartrate-resistant acid phosphatase method (TRAP) and analysed for osteoclasts and bone resorption. Black arrows indicate bone resorption. Pictures 1-4 (Trehalose group) demonstrate strong bone resorption at sites of osteoclast staining. Pictures 2 and 4 show bone resorption of compact bone of the diaphysis and pictures 1 and 3 resorption of epiphyseal bone underlying the articular surfaces. Bone from EndoMTX treated animals (pictures 5-8) do not show bone reorption of compact bone of the diaphysis or epiphyseal bone.

### Example 13: Assessment of therapeutic effects of EndoMTX on serum levels of pro-inflammatory cytokines in collagen induced arthritis (CIA) in DBA/1 mice

Male DBA/1 mice with an age of ten weeks at arrival were purchased from Taconic Europe and housed in isolated cages under save environmental conditions (five mice per cage, 22°C, 30-70 % humidity and 12 h light/dark cycle) with food and water ad libitum. Experimental design was reviewed and approved by local government.

Mice were assigned to the experimental groups acclimated at least 7 days before induction of collagen induced arthritis. Animals were anesthetised intraperitoneally (i.p.) with a mixture of Xylazin and Ketamin. Each animal obtained 4 ml/kg as application volume. 0.5 ml Rompun (20 mg/ml Xylazin) and 1 ml Ketamin (100 mg/ml Ketamin) were mixed (1:2) and diluted 1:4 in 4.5 ml 0.9 % NaCl. 2. For the collagen Type II-CFA emulsions, collagen (bovine, 4 mg/ml) was mixed 1:1 with CFA by repeated passage through a flexible tube connecting two 1-ml syringes (syringe-syringe-method). This mixture was kept on ice and was applied within 4 hours after preparation. For the immunisation 100 µl Collagen/CFA-emulsion was injected subcutaneously at the base of the tail. The injection of Collagen/CFA-emulsion was done once on day 0 and once as a booster immunisation on day 21.

Treatment with drugs started day 15. Drugs were given three times a week (Mo, Wed, Fri) for the following three weeks. EndoMTX (10 mM DOTAP; 1.1 mM methotrexate) was prepared by co-extrusion as described in Example 10. Drug solutions were administered i.v. with slow bolus in a volume of 10 ml/kg. Animals of each group were treated according to the table.

| **Group name** | **Animal no.** | **Treatment** | **Appl. vol. [ml/kg]** | **Number of appl. per week** | **Dose [mg/kg]** |
|---|---|---|---|---|---|
| Disease control | 15 | 9.8 % Trehalose | 10 | 3 | -- |
| MTX | 15 | Methotrexate | 10 | 3 | 1,0 |
| EndoMTX | 15 | EndoMTX | 10 | 3 | 1,0* |

| | | | | | |
|---|---|---|---|---|---|
| * given as methotrexate concentration | | | | | |

The cytokines IL-6 and KC (IL-8 homologue) were analysed using the Becton Dickinson CBA Soluble Protein Flex Set according to the manufacturer's instructions. 50 µL of standards or freshly thawed sera, which were diluted 1:4 with assay diluent, were mixed with 50 µL of capture bead solution (for 100 assays: 100 µl of IL-6 capture beads + 100 µl of KC capture beads + 4800 µl of capture bead diluent) for 5 min at 500 rpm and incubated for 1h at RT in the dark. This was followed by adding 50 µl of detection reagent solution (for 100 assays: 100 µl of IL-6 detection reagent + 100 µl of KC detection reagent + 4800 µl of detection reagent diluent). The solution was mixed for 5 min at 500 rpm and incubated for 1 h at RT in the dark. After washing, beads were resuspended in 150 µl of washing buffer and immediately measured with the FACSCalibur and CellQuest software and analysed by the FCAP array software v1.0. The detection range of mouse IL-6 was 6.5 pg/ml to 5000 pg/ml and of mouse KC 16.2 pg/ml to 5000 pg/ml.

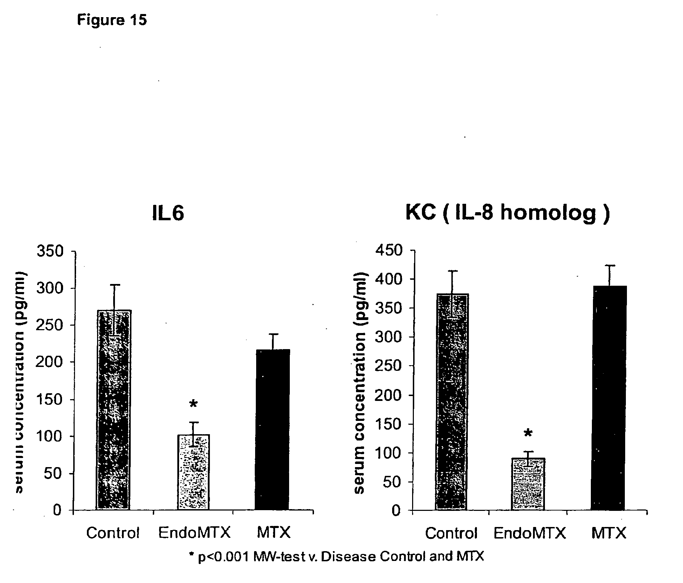

IL-6 is produced at the site of inflammation and plays a key role in the acute phase response (Gabay, 2006 # 25). Healthy DBA/1 mice revealed a mean serum IL-6 level of 35 pg/ml, whereby CIA mice in the control group (Trehalose) showed IL-6 levels between 77 pg/ml and 460 pg/ml with a mean level of 271 pg/ml. As shown in figure 15 only treatment with EndoMTX significantly reduced the IL-6 serum levels up to 67 % compared to the control group.

The Keratinocyte Cytokine KC (CXCL1) is involved in chemotaxis and cell activation of neutrophils and is designated as the murine Interleukin-8 homologue (Bozic et al., 1994 # 26). Serum KC levels in healthy control DBA/1 mice were between 90 pg/ml and 177 pg/ml with a mean value of 133 pg/ml and in the CIA DBA/1 mice on day 39 (Trehalose group) 237 pg/ml and 790 pg/ml with a mean value of 374 pg/ml. As shown in Figure 15 only treatment with EndoMTX significantly reduced the KC serum levels up to 77 % compared to the control group.

### Example 14: Therapeutic effects of DOTAP liposomes on rat Carrageenan-induced paw inflammation

Male Sprague Dawley rats with an average weight of 297 grams at arrival were purchased from Harlan Inc. and housed in isolated cages under save environmental conditions (3-4 rats per cage, 22°C, 30-70 % humidity and 12 h light/dark cycle) with food and water ad libitum. Animals were acclimated for 3 days prior to being placed on study. Experimental design was reviewed and approved by local government.

10 mM DOTAP/DOPC liposomal preparations with a molar ratio of 100/0, 75/25, 50/50, 25/75 and 5/95 mol% of the two lipids (total volume of 40 ml) were prepared by the lipid film method as described in WO 2004/002468 by Mundus et al., rehydration was performed with 10 % trehalose. The resulting solution was extruded 5 times through a polycarbonate membrane with a pore size of 200 nm.

Animals (6/group), dosed with test articles by intravenous injection (5 ml/kg) into the tail vein, were injected with 100 µl of 1.2 % carrageenan into the right hind footpad and were then euthanized at four hours post injection for evaluation of paw swelling, based on paw weight determination. Test article solutions were administered i.v. with slow bolus. Animals were dosed 30 minutes post Carrageenan injection as indicated below:

| **Group name** | **Animal no.** | **Treatment** | **Appl. vol. [ml/kg]** |
|---|---|---|---|
| Trehalose | 6 | 9.8 % Trehalose | 5 |
| D100/0 | 6 | DOTAP/DOPC 100/0 mol% | 5 |
| D75/25 | 6 | DOTAP/DOPC 75/25 mol% | 5 |
| D50/50 | 6 | DOTAP/DOPC 50/50 mol% | 5 |
| D25/75 | 6 | DOTAP/DOPC 25/75 mol% | 5 |
| D5/95 | 6 | DOTAP/DOPC 5/95 mol% | 5 |

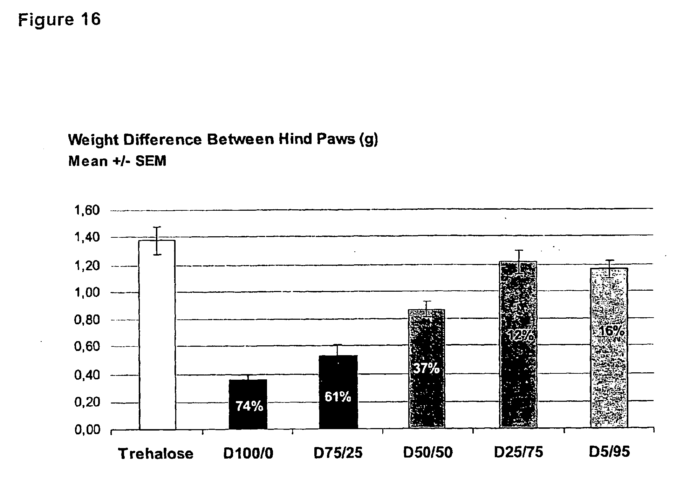

Figure 16 shows that the treatment with D100/0, 75/25, or 50/50 significantly and dose-responsively decreased the difference in weight between the right and left paws (74, 61, and 37%, respectively). Results indicate that only treatment with cationic DOTAP liposomes with higher relative DOTAP levels/cationic charge (100/0, 95/5P, 75/25, 50/50) significantly reduced paw swelling associated with carrageenan injection compared to saline treatment.

### Example 15: In vivo binding kinetics Methotrexate loaded cationic lipid complexes in the vasculature of solid tumors

10 mM DOTAP/Rh-DOPE liposomes with a molar ratio 95/5 mol% (total volume of 10 ml) were prepared by the lipid film method, rehydration was performed with 10% trehalose. The resulting solution (theoretical concentration 9.5 mM DOTAP, 0.5 mM Rh-DOPE) was extruded 5 times through a polycarbonate membrane with a pore size of 200 nm.

DOTAP/Rh-DOPE + MTX liposomes were prepared by first obtaining 20 mM DOTAP/Rh-DOPE liposomes with a molar ratio of 95/5 mol% (total volume of 5 ml) by the lipid film method, rehydration was performed with 10% trehalose. Next, the liposomes were mixed with 5 ml of a sodium MTX solution (2.2 mM, prepared from diluting a 220 mM sodium MTX solution with 10% trehalose). The resulting solution (theoretical concentration now 9.5 mM DOTAP, 0.5 mM Rh-DOPE, 1.1 mM MTX) was extruded 5 times through a polycarbonate membrane with a pore size of 200 nm.

Experiments were carried out as described by Krasnici et al. {Kransnici, 2003 #22} using male Syrian golden hamsters (60-70 g b.w.) purchased from Charles River according to institutional and governmental guidelines. The animals were housed in single cages and had free access to tap water and standard laboratory food throughout the experiments.

To permit quantitative fluorescence analysis of striated muscle and tumor, a dorsal skinfold chamber preparation consisting of 2 symmetrical titanium frames was surgically implanted as described earlier in detail. The chambers were well tolerated and animals exhibited no signs of discomfort. All surgical procedures were performed under anesthesia with ketamine (100 mg/kg b.w. i.p., Ketavet; Parke-Davis, Berlin, Germany) and xylazine (10 mg/kg b.w. i.p, Rompun; Bayer, Leverkusen, Germany). Permanently indwelling fine polyethylene catheters (PE10, inner diameter 0.28 mm) were implanted into the right jugular vein 24 hr prior to fluorescent macroscopy. Only chambers fulfilling inclusion criteria of intact microcirculation with no signs of inflammation have been included in experiments. A-mel 3 tumors have been allowed to grow in the chamber for 7 days prior to experiments were performed.

For detection of rhodamine-liposomes-induced fluorescence, the awake chamber-bearing hamster was immobilized in a Perspex tube on a specially designed stage (Effenberger, Munich, Germany) under a Leitz macroscope (Type 307-143003/514660, Leitz, Munich, Germany). Autofluorescence of chamber tissue was determined by intravital macroscopy prior to intravenous injection of liposomes and subtracted from the respective fluorescence images. At 0.5, 1, 2, 3, 5, 10, 15, 20, 30, 45, 60, 120,180, 240 min after injection, fluorescence was measured within the chamber preparation. Fluorophores were excited at 530-560 nm for 2 sec (100 W, HBO mercury lamp). Emission fluorescence was detected above 580 nm and acquired by a silicon-intensified target video camera (C2400-08, Hamamatsu, Herrsching, Germany). The camera setup was previously calibrated for linearity. Fluorescence images were digitized on-line by an image analysis system (KS400, Zeiss, Germany) and stored on hard disk. Spatial inhomogeneities of camera or illumination were compensated by shading correction. Densitometrical measurement of fluorescence intensities was performed off-line after correction for background fluorescence by subtraction of tissue autofluorescence. Fluorescence values are given in percentages of the solid reference fluorescence signal (% standard) inserted into each chamber (ImpregumF; ESPE, Seefeld, Germany). The geometric resolution of all images was 512 x 512 pixels at a total densitometric resolution of 255 gray values. ROI were selected and set in the epi-illumination image of each chamber preparation prior to injection of fluorescently labeled liposomes.

Hamsters were assigned to 2 experimental groups. In each experiment 5 µl/g b.w. of the respective liposome formulation has been injected i.v. and fluorescence macroscopy has been conducted as described above.

Comparison of tumor and normal tissue fluorescence intensities has been performed using Wilcoxon Signed Rank Test. Area below fluorescence time curves have been calculated by SigmaPlot software.
Hamsters were assigned to the experimental groups of 6 animals and injected with:

| **group name** | **formulation** | **number of experiments** |
|---|---|---|
| **EndoMTX** | DOTAP/Rh-DOPE 95/5 +MTX | 6 |
| **EndoMTX placebo** | DOTAP/Rh-DOPE 95/5 | 6 |

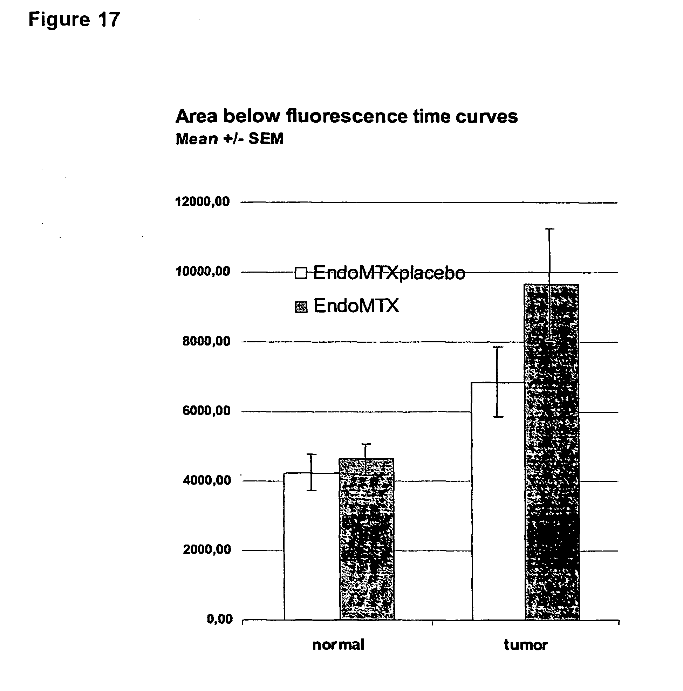

Both formulations were well tolerated by animals and revealed *in vivo* targeting properties of tumor microvessels indicated by higher fluorescence values measured in tumor in comparison to normal surrounding host tissue (Figure 17). Although both formulations show a similar binding capacity to normal microvessels ratio DOTAP-Rh-DOPE-MTX lipid complexes showed a higher binding capacity to tumor microvessels compared to DOTAP/Rh-DOPE lipid complexes without MTX.

### Example 16: Therapeutic effect of EndoTAG-1 compared to EndoTAG-1 placebo + Taxol^{®} on collagen induced arthritis in B10RIII mice

The experiment was carried out according to the procedures described in Example 6 with the following dosing schedule:
Treatment with drugs started when animals showed first clinical signs of arthritis (at least one paw with arthritis score>0). From this day of enrollment (day 0) drugs were given every second day until day 11 and clinical symptoms were monitored daily. Drugs were prepared as described in Example 4. Drug solutions were administered iv with slow bolus in a volume of 10ul/g. Taxol^{®} was applied 15 min after the EndoTAG-1 placebo. Animals of each group were treated according to the table.

| **Group name** | **Animal no.** | **Treatment** | **Appl. vol. [ml/kg]** | **Dose [mg/kg]** |
|---|---|---|---|---|
| Disease control | 15 | 9.8 % Trehalose | 10 | -- |
| EndoTAG-1 placebo + Taxol | 15 | EndoTAG-1 placebo + Taxol^{#} | 10 | 2,56* |
| EndoTAG-1 q2d | 15 | EndoTAG-1 | 10 | 2,56* |

| | | | | |
|---|---|---|---|---|
| * given as paclitaxel concentration # Taxol was applied 15 min after the EndoTAG-1 placebo | | | | |

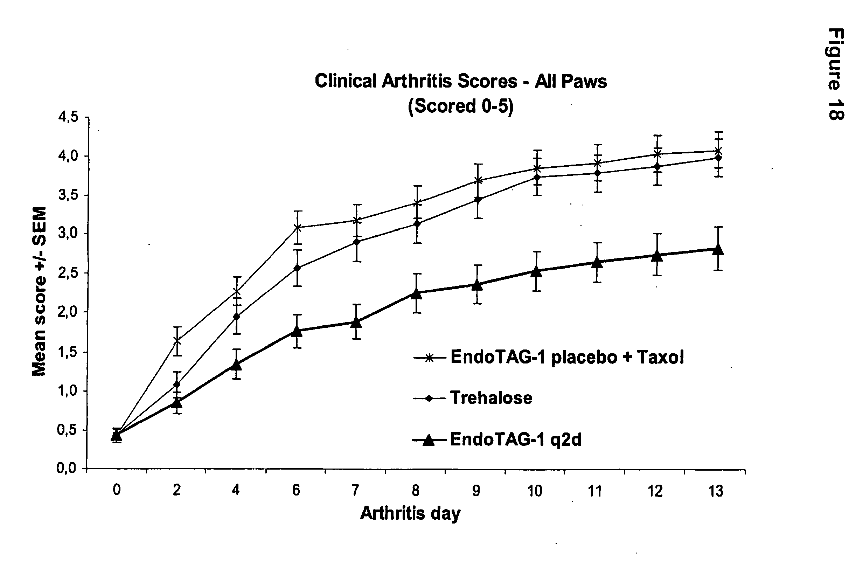

As shown in Figure 18. EndoTAG-1 showed a clear efficacy in the therapeutic treatment of arthritic paws when compared to the trehalose disease group, measured as a decrease in the mean clinical arthritis score. This effect is observed from day 6 after enrollment on and was visible until the end of the study at day 13. These effects were dependent upon the liposomal formulation used for EndoTAG-1, as no efficacy was observed for the treatment with a combination of EndoTAG-1 placebo and Taxol^{®}.

### Example 17: Assessment of therapeutic effects of EndoMTX at low dose on collagen induced arthritis in DBA/1 mice

The experiment was carried out as described in Example 8 but with the following groups and dosing schedule (EndoMTX and Methotrexate stock solutions were diluted to obtain a lower dose):

| **Group name** | **Animal no.** | **Treatment** | **Appl. vol. [ml/kg]** | **Number of appl. per week** | **Dose [mg/kg]** |
|---|---|---|---|---|---|
| Disease control | 13 | 9.8% Trehalose | 10 | 3 | -- |
| MTX | 13 | Methotrexate | 10 | 3 | 0.5 |
| EndoMTX | 13 | EndoMTX | 10 | 3 | 0.5* |

| | | | | | |
|---|---|---|---|---|---|
| * given as methotrexate concentration | | | | | |

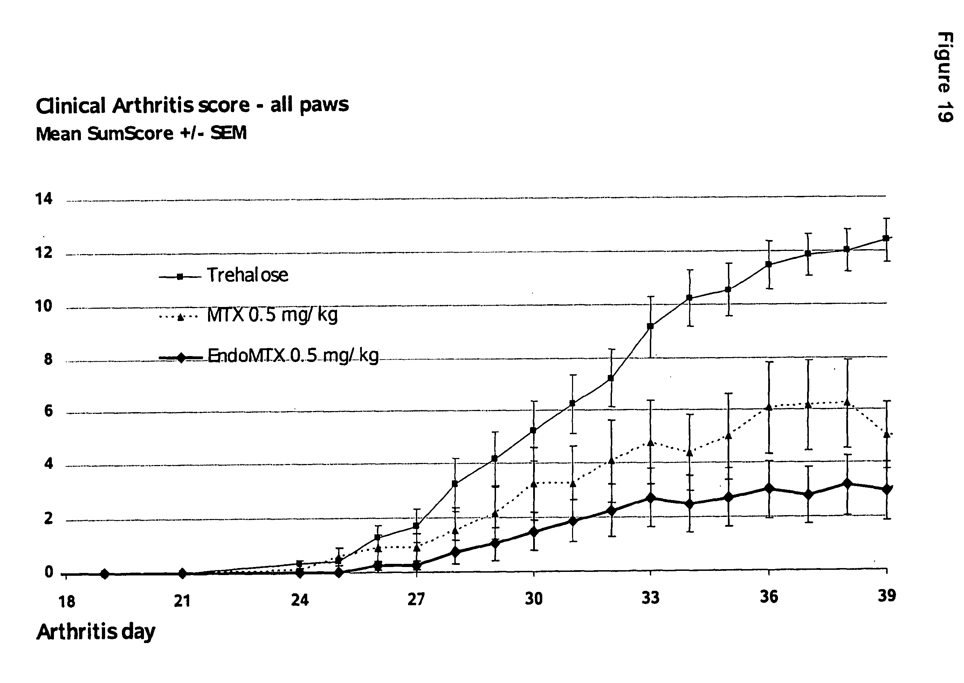

As shown in Figure 19 EndoMTX administered at low doses of 0.5mg/kg showed a clear efficacy in the treatment of the arthritic paws when compared to the trehalose disease group, measured as a decrease in the mean clinical arthritis sum score. This effect is observed from day 25 on and was visible until the end of the study. EndoMTX was more effective then the free Methotrexate throughout most of the study period.

### Example 18: Therapeutic effect of EndoTAG-1 + Enbrel® co-treatment on collagen induced arthritis in B10RIII mice

Male B10RIII mice with an age between 7-9 weeks at arrival were purchased from Jackson Laboratories and housed in isolated cages under save environmental conditions (4-5 mice per cage, 22°C, 30-70% humidity and 12h light/dark cycle) with food and water ad libitum. Experimental design was reviewed and approved by local government.

Animals were anesthetized with Isoflurane and injected with 150 µl of Freund's Complete Adjuvant (Difco) containing bovine type II collagen (Elastin Products, Owensville, MO) (2 mg/ml) and supplemental Mycobacterium tuberculosis H37 RA (Difco) at the base of the tail on days 0 and 15. Male B10RIII mice with developing type II collagen arthritis were randomized into groups that received once daily i.v. treatments.

After two weeks the animals were controlled for symptoms of collagen induced arthritis (CIA) by joint scoring every day. To document the progression of arthritis for each animal, grades of arthritis were classified as arthritis index (AI) by score 0-5 per joint. Symptoms were defined by the following symptom-list:
0=normal
1=1 hind or fore paw joint affected or minimal diffuse erythema and swelling
2=2 hind or fore paw joints affected or mild diffuse erythema and swelling
3=3 hind or fore paw joints affected or moderate diffuse erythema and swelling
4= Marked diffuse erythema and swelling, or =4 digit joints affected)
5=Severe diffuse erythema and severe swelling entire paw, unable to flex digits)

Treatment with drugs started when animals showed first clinical signs of arthritis (at least one paw with arthritis score>0). From this day of enrollment (day 0) drugs were given every second day until day 11 and clinical symptoms were monitored daily. EndoTag-1 (DOTAP 50%/DOPC47%/paclitaxel 3%) or EndoTAG-placebo (DOTAP 50%/DOPC 50%) have a lipid content of 10 mM in a 10% m/m trehalose dihydrate solution and were prepared as described in WO 2004/002468 by Mundus et al.. Enbrel^{®} (Etanercept) is a dimeric fusion protein consisting of the extracellular ligand-binding portion of the human 75 kilodalton (p75) tumor necrosis factor receptor (TNFR) linked to the Fc portion of human IgG1 and was purchased from AMGEN. Drug solutions were administered i.v. with slow bolus in a volume of 10ul/g. Animals of each group were treated according to the table.

| **Group name** | **Animal No.** | **Treatment** | **Appl. vol. [ml/kg]** | **Dose mg/kg** |
|---|---|---|---|---|
| Disease control | 15 | 9.8 % Trehalose | 10 | -- |
| EndoTAG-1 q2d | 15 | EndoTAG-1 | 10 | 2,56* |
| Enbrel 10mg/kg | 10 | Etanercept | 10 | 10 |
| EndoTAG-1 q2d +Enbrel 10mg/kg | 15 | EndoTAG-1 q2d +Enbrel 10mg/kg | 10 | 2,56* |
| Enbrel | 10 | Etanercept | 10 | 10 |

| | | | | |
|---|---|---|---|---|
| * given as paclitaxel concentration | | | | |

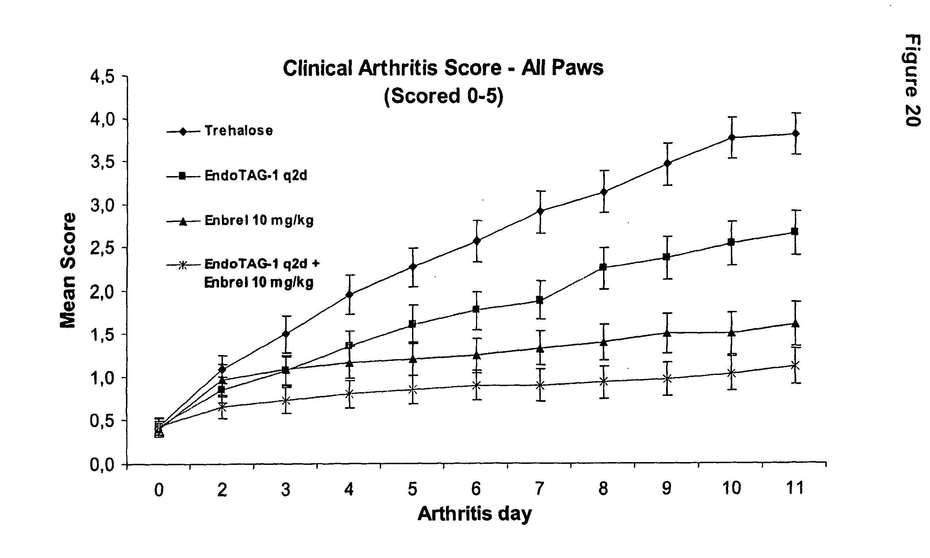

As shown in Figure 20 a combination of EndoTAG-1 and Enbrel showed a clear efficacy in the therapeutic treatment of arthritic paws when compared to the trehalose disease group. This effect was higher compared to the single treatment with EndoTAG-1 or Enbrel®.

### Example 19: Treatment of a human patient with EndoMTX

A human patient suffering from rheumatoid arthritis is treated with a pharmaceutical preparation comprising EndoMTX (10 mM DOTAP; 1,1 mM methotrexate) which is prepared as described afore. Besides EndoMTX the pharmaceutical preparation may comprise pharmaceutical acceptable carriers, buffers or other additives.

The pharmaceutical preparation is administered intravenously to the human patient bolus injection or by infusion over a longer period of time depending on the dose and volume administered.

The pharmaceutical preparation is administered at a single dose of MTX between 2.5 mg/kg and 30 mg/kg, preferably about 7.5 mg/kg. This dose is applied to the patient at a frequency between once a week and once a month. In the event that drug related toxicity is observed the treatment is abandoned.

The treatment with EndoMTX can be combined with the treatment by other DMARDs especially with treatment by TNF targeting biologics.

### References

1. Gabriel, S.E., The epidemiology of rheumatoid arthritis. Rheum Dis Clin North Am, 2001. 27(2): p. 269-81.
2. Smolen, J.S. and G. Steiner, Therapeutic strategies for rheumatoid arthritis. Nat Rev Drug Discov, 2003. 2(6): p. 473-88.
3. Steiner, G., et al., Cytokine production by synovial T cells in rheumatoid arthritis. Rheumatology (Oxford), 1999. 38(3): p. 202-13.
4. Stout, R.D., Macrophage activation by T cells: cognate and non-cognate signals. Curr Opin Immunol, 1993. 5(3): p. 398-403.
5. Paleolog, E.M., Angiogenesis in rheumatoid arthritis. Arthritis Res, 2002. 4 Suppl 3: p. S81-90.
6. Koch, A.E., The role of angiogenesis in rheumatoid arthritis: recent developments. Ann Rheum Dis, 2000. 59 Suppl 1: p. i65-71.
7. Cutolo, M., et al., Anti-inflammatory mechanisms of methotrexate in rheumatoid arthritis. Ann Rheum Dis, 2001. 60(8): p. 729-35.
8. Borchers, A.T., et al., The use of methotrexate in rheumatoid arthritis. Semin Arthritis Rheum, 2004. 34(1): p. 465-83.
9. Szekanecz, Z. and A.E. Koch, Cell-cell interactions in synovitis. Endothelial cells and immune cell migration. Arthritis Res, 2000. 2(5): p. 368-73.
10. Wilder, R.L., Integrin alpha V beta 3 as a target for treatment of rheumatoid arthritis and related rheumatic diseases. Ann Rheum Dis, 2002. 61 Suppl 2: p. ii96-9.
11. Miller, W.H., et al., Identification and in vivo efficacy of small-molecule antagonists of integrin alphavbeta3 (the vitronectin receptor). Drug Discov Today, 2000. 5(9): p. 397-408.
12. Badger, A.M., et al., Disease-modifying activity of SB 273005, an orally active, nonpeptide alphavbeta3 (vitronectin receptor) antagonist, in rat adjuvant-induced arthritis. Arthritis Rheum, 2001. 44 (1): p. 128-37.
13. Garrood, T. and C. Pitzalis, Targeting the inflamed synovium: The quest for specificity. Arthritis Rheum, 2006. 54(4): p. 1055-60.
14. Trif, M., et al., Liposomes as possible carriers for lactoferrin in the local treatment of inflammatory diseases. Exp Biol Med (Maywood), 2001. 226(6): p. 559-64.
15. Cruz, M.E., et al., Liposomal superoxide dismutases and their use in the treatment of experimental arthritis. Methods Enzymol, 2005. 391: p. 395-413.
16. Patel, K.R. and J.D. Baldeschwieler, Mouse Lewis lung carcinoma and hepatoma ascites treatment by combination of liposome chemotherapy and non-specific immunotherapy. Int J Cancer, 1984. 34(5): p. 717-23.
17. Pignatello, R., et al., Effect of liposomal delivery on in vitro antitumor activity of lipophilic conjugates of methotrexate with lipoamino acids. Drug Deliv, 2003. 10(2): p. 95-100.
18. Kim, M.M., et al., Pharmacokinetics of methotrexate after intravenous and intramuscular injection of methotrexate-bearing positively charged liposomes to rats. Biopharm Drug Dispos, 1995. 16(4): p. 279-93.
19. Brackertz, D., G.F. Mitchell, and I.R. Mackay, Antigen-induced arthritis in mice. I. Induction of arthritis in various strains of mice. Arthritis Rheum, 1977. 20(3): p. 841-50.
20. Veihelmann, A., et al., Inhibition of nitric oxide synthesis improves detoxication in inflammatory liver dysfunction in vivo. Am J Physiol, 1997. 273(2 Pt 1): p. G530-6.
21. Haris, K., et al., Coronary artery skeleton detection based on topographic features. Stud Health Technol Inform, 1997. 43 Pt B: p. 512-6.
22. Krasnici, S., et al., Effect of the surface charge of liposomes on their uptake by angiogenic tumor vessels. Int J Cancer, 2003. 105(4): p. 561-7.
23. Asaishi, K., et al., Quantitative analysis of microvascular structure and function in the amelanotic melanoma A-Mel-3. Cancer Res, 1981. 41 (5): p. 1898-904. microvascular studies in unanesthetized hamsters. Res Exp Med (Berl), 1980. 177(2): p. 125-34.
25. Gabay C. (2006), Arthritis Research & Therapy, 8 (Suppl. 2), p.3.
26. Bozic CR et al. (1994) J Biol. Chem. 269, 29355.

## Claims

1. Use of a cationic liposomal preparation comprising at least one small molecule or polypeptide active agent, and having a positive zeta potential for the manufacture of a medicament for the prevention and/or inhibition of cartilage and/or bone erosion in the course of an inflammatory and/or autoimmune disorder.

2. The use of claim 1, wherein said disorder is rheumatoid arthritis.

3. The use of claim 1 or 2, wherein said cationic liposomal preparation comprises a cationic lipid, optionally at least one further amphiphile and optionally at least one stabilizing agent.

4. The use of claim 3, wherein said cationic liposomal preparation comprises a cationic lipid in an amount of at least about 30 mol%, and optionally at least one further amphiphile in an amount of up to about 70 mol%.

5. The use of claim 3 or 4, wherein said further amphiphile is a neutral and/or anionic lipid, particularly a phosphoethanolamine (PE) and/or phosphocholine (PC) such as DOPE and/or DOPC.

6. The use of any one of claims 3-5, wherein said cationic lipid is DOTAP, DSTAP or DPTAP.

7. The use of claim 6, wherein said neutral and/or anionic lipid is a pegylated lipid such as pegylated DOPE, DSPE and/or DOPC.

8. The use of any one of claims 1-7, wherein said active agent is a cytotoxic or cytostatic agent.

9. The use of any one of claims 1-7, wherein said active agent is a non-steroidal anti-inflammatory drug (NSAID) or a disease-modifying anti-rheumatic drug (DMARD).

10. The use of claim 9, wherein said disease-modifying anti-rheumatic drug is an antifolate.

11. The use of claim 10, wherein said antifolate is methotrexate.

12. The use of claim 9, wherein said disease-modifying anti-rheumatic drug is selected from doxycycline, chondroitin sulfate, leflunomide, sulphasalazine, penicillamine, gold salts like aurothiomalate, cyclophosphamide, azathioprine, cyclosporine A, minocycline, glucocorticoids and antimalaria drugs.

13. The use of claim 9, wherein said disease-modifying anti-rheumatic drug is an RGD peptide.

14. The use of claim 8, wherein said cytotoxic or cytostatic agent is a taxane.

## Patentansprüche

1. Verwendung einer kationischen liposomalen Zubereitung, die mindestens einen niedermolekularen oder einen Polypeptid Wirkstoff umfasst und die ein positives Zetapotential besitzt zur Herstellung eines Medikaments zur Vorbeugung und/oder Hemmung einer Knorpel- und/oder Knochenabnutzung im Verlauf einer Entzündungs- und/oderAutoimmunerkrankung.

2. Verwendung nach Anspruch 1, in der die Erkrankung rheumatoide Arthritis ist.

3. Verwendung nach Anspruch 1 oder 2, in der die kationische liposomale Zubereitung ein kationisches Lipid, gegebenenfalls mindestens ein weiteres Amphiphil und gegebenenfalls mindestens ein Stabilisierungsmittel umfasst.

4. Verwendung nach Anspruch 3, in der die kationische liposomale Zubereitung ein kationisches Lipid in einer Menge von mindestens etwa 30 Mol% und gegebenenfalls ein weiteres Amphiphil in einer Menge von bis zu etwa 70 Mol% umfasst.

5. Verwendung nach Anspruch 3 oder 4, in der das weitere Amphiphil ein neutrales und/oder anionisches Lipid ist, insbesondere ein Phosphoethanolamin (PE) und/oder Phosphocholin (PC) wie DOPE und/oder DOPC.

6. Verwendung nach einem der Ansprüche 3-5, in der das kationische Lipid DOTAP, DSTAP oder DPTAP ist.

7. Verwendung nach Anspruch 6, in der das neutrale und/oder anionische Lipid ein pegyliertes Lipid wie pegyliertes DOPE, DSPE und/oder DOPC ist.

8. Verwendung nach einem der Ansprüche 1-7, in der der Wirkstoff ein zytotoxischer oder zytostatischer Wirkstoff ist.

9. Verwendung nach einem der Ansprüche 1-7, in der der Wirkstoff ein nichtsteroidales entzündungshemmendes Arzneimittel (NSAID) oder ein krankheitsmodifizierendes Antirheumatikum (DMARD) ist.

10. Verwendung nach Anspruch 9, in der das krankheitsmodifizierende Antirheumatikum ein Antifolat ist.

11. Verwendung nach Anspruch 10, in der das Antifolat Methotrexat ist.

12. Verwendung nach Anspruch 9, in der das krankheitsmodifizierende Antirheumatikum aus Doxycyclin, Chondroitinsulfat, Leflunomid, Sulphasalazin, Penicillamin, Goldsalzen wie Aurothiomalat, Cyclophosphamid, Azathioprin, Cyclosporin A, Minocyclin, Glucocorticoiden und Antimalariamitteln ausgewählt ist.

13. Verwendung nach Anspruch 9, in der das krankheitsmodifizierende Antirheumatikum ein RGD Peptid ist.

14. Verwendung nach Anspruch 8, in der der zytotoxische oder zytostatische Wirkstoff ein Taxan ist.

## Revendications

1. Utilisation d'une préparation liposomale cationique, comprenant au moins une petite molécule ou un agent actif polypeptidique et possédant un potentiel zéta positif, pour la fabrication d'un médicament destiné à la prévention et/ou l'inhibition de l'érosion du cartilage et/ou de l'os au cours d'un trouble inflammatoire et/ou autoimmunitaire.

2. Utilisation selon la revendication 1, dans laquelle ledit trouble est la polyarthrite rhumatoïde.

3. Utilisation selon la revendication 1 ou 2, dans laquelle ladite préparation liposomale cationique comprend un lipide cationique, facultativement au moins un amphiphile supplémentaire et facultativement au moins un agent de stabilisation.

4. Utilisation selon la revendication 3, dans laquelle ladite préparation liposomale cationique comprend un lipide cationique en une quantité d'au moins environ 30 % en mole, et facultativement au moins un amphiphile supplémentaire en une quantité allant jusqu'à environ 70 % en mole.

5. Utilisation selon la revendication 3 ou 4, dans laquelle ledit amphiphile supplémentaire est un lipide neutre et/ou anionique, en particulier une phosphoéthanolamine (PE) et/ou une phosphocholine (PC), comme la DOPE et/ou la DOPC.

6. Utilisation selon l'une quelconque des revendications 3 à 5, dans laquelle ledit lipide cationique est le DOTAP, le DSTAP ou le DPTAP.

7. Utilisation selon la revendication 6, dans laquelle ledit lipide neutre et/ou anionique est un lipide pégylé, comme la DOPE pégylée, la DSPE pégylée et/ou la DOPC pégylée.

8. Utilisation selon l'une quelconque des revendications 1 à 7, dans laquelle ledit agent actif est un agent cytotoxique ou cytostatique.

9. Utilisation selon l'une quelconque des revendications 1 à 7, dans laquelle ledit agent actif est un médicament anti-inflammatoire non stéroïdien (AINS) ou un médicament antirhumatismal modificateur de la maladie (ARMM).

10. Utilisation selon la revendication 9, dans laquelle ledit médicament antirhumatismal modificateur de la maladie est un antifolate.

11. Utilisation selon la revendication 10, dans laquelle ledit antifolate est le méthotréxate.

12. Utilisation selon la revendication 9, dans laquelle ledit médicament antirhumatismal modificateur de la maladie est choisi parmi la doxycycline, la chondroïtine sulfate, le leflunomide, la sulphasalazine, la pénicillamine, les sels d'or tels que l'aurothiomalate, le cyclophosphamide, l'azathioprine, la cyclosporine A, la minocycline, les glucocorticoïdes et les antipaludiques.

13. Utilisation selon la revendication 9, dans laquelle ledit médicament antirhumatismal modificateur de la maladie est un peptide RGD.

14. Utilisation selon la revendication 8, dans laquelle ledit agent cytotoxique ou cytostatique est un taxane.
